(19) Europäisches Patentamt
European Patent Office
Office européen des brevets

(11) Publication number: 0 121 341
B1

(12) EUROPEAN PATENT SPECIFICATION

(45) Date of publication of patent specification: 17.11.88

(51) Int. Cl.[4]: C 07 D 487/04, A 61 K 31/505

(21) Application number: 84301383.0

(22) Date of filing: 02.03.84

# (54) Triazolo(4,3-c)pyrimidines and triazolo(1,5-c)pyrimidines substituted by nitrogen-containing heterocyclic rings.

(30) Priority: 03.03.83 US 471836
03.03.83 US 471837

(43) Date of publication of application:
10.10.84 Bulletin 84/41

(45) Publication of the grant of the patent:
17.11.88 Bulletin 88/46

(84) Designated Contracting States:
AT BE CH DE FR GB IT LI NL SE

(56) References cited:
AT-B- 303 036
DE-A-3 029 871
DE-B-1 074 589
FR-A-1 205 144
US-A-4 269 980

(73) Proprietor: RIKER LABORATORIES, INCORPORATED
3M Center - 225-5S-01
Saint Paul Minnesota 55144 (US)

(72) Inventor: Wade, James J.
2501 Hudson Road P.O. Box 33427
Saint Paul Minnesota 55133 (US)

(74) Representative: Baillie, Iain Cameron et al
c/o Ladas & Parry Isartorplatz 5
D-8000 München 2 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 121 341 B1

Courier Press, Leamington Spa, England.

**Description**

Technical Field

The present invention relates to triazolo[4,3-c]pyrimidines and triazolo[1,5-c]pyrimidines, and more specifically to 1,2,4-triazolo[4,3-c]pyrimidines and 1,2,4-triazolo[1,5-c]pyrimidines. The pharmacological use of the compounds of the invention as bronchodilators, and pharmaceutical compositions comprising the compound are also within the scope of the invention.

Background of the Invention

Some 1,2,4-triazolo[4,3-c]pyrimidines and 1,2,4-triazolo[1,5-c]pyrimidines are known in the art. Certain 1,2,4-triazolo[4,3-c]pyrimidines and 1,2,4-triazolo[1,5-c]pyrimidines are disclosed as being bronchodilators in the patents discussed below, the 1,2,4-triazolo[1,5-6] pyrimidines being referred to therein as triazolo-[2,3-c]pyrimidines:

United Kingdom Patent No. 859,287 discloses what were believed to be the compounds 3-amino-7-methyl-5-methylthio-1,2,4-triazolo[4,3-c]pyrimidine and 3-amino-7-chloro-5-methyl-1,2,4-triazolo[4,3-c]-pyrimidine. United Kingdom Patent No. 859,287 also discloses 2-amino-1,2,4-triazolo[1,5-c]pyrimidines which are substituted on the pyrimidine ring at the 5, 7 and 8 positions by certain combinations of substituents selected from hydrogen, alkyl, halogen-substituted alkyl, hydroxy-substituted alkyl, alkoxy-substituted alkyl, alkenyl, cycloalkyl, amino, alkylamino, dialkylamino, phenyl, alkylthio, alkoxy and halogen substituents.

United Kingdom Patent No. 898,408 discloses 3-amino-1,2,4-triazolo[4,3-c]pyrimidines which are substituted on the pyrimidine ring at the 5-position by an alkyl, alkylthio, or amino substituent, at the 7-position by an alkyl, halogen-substituted alkyl or halogen substituent, and at the 8-position by hydrogen or an alkyl or alkenyl substituent.

United Kingdom Patent No. 873,223 discloses 2-amino or 2-acetamido-1,2,4-triazolo[1,5-c]pyrimidines which are substituted on the pyrimidine ring at the 5, 7 and 8 positions by certain combinations of substituents selected from hydrogen, alkyl, halogen-substituted alkyl, alkoxy-substituted alkyl, alkenyl, cycloalkyl, alkylthio and halogen substituents.

United Kingdom Patent No. 897,870 discloses 2-alkylamino-1,2,4-triazolo[1,5-c]pyrimidines, 2-dialkylamino-1,2,4-triazolo[1,5-c]pyrimidines, and 1,2,4-triazolo[1,5-c]pyrimidines containing a piperidino or morpholino substituent bonded at the 2-position through the nitrogen atom, which compounds are substituted on the pyrimidine ring at the 5, 7 and 8 positions by certain combinations of substituents selected from hydrogen, alkyl, halogen-substituted alkyl, hydroxy-substituted alkyl, alkenyl and halogen substituents.

The following related articles disclose the synthesis of certain 1,2,4-triazolo[4,3-c]pyrimidines and certain 1,2,4-triazolo[1,5-c]pyrimidines as potential bronochodilators:

G. W. Miller et al., *J. Chem. Soc.,* 1963, 5642, discloses 1,2,4-triazolo[4,3-c]pyrimidines which are substituted at the 3-position by amino or imino substituents, and on the pyrimidine ring by alkyl substituents or alkyl and alkenyl substituents. This publication also discloses 2-amino- or 2-acetamido-1,2,4-triazolo[1,5-c]pyrimidines (referred to therein as triazolo[2,3-c]pyrimidines) which are substituted on the pyrimidine ring by, for example, hydrogen and alkyl substituents. Certain of these latter compounds are said to be bronchodilators.

G. W. Miller et al., *J. Chem. Soc., 1965,* 3357, discloses the compound 3-hydroxy-7-methyl-5-n-propyl-1,2,4-triazolo[4,3-c]pyrimidine. This publication also discloses 1,2,4-triazolo[1,5-c]pyrimidines (referred to therein as triazolo[2,3-c]pyrimidines) which are substituted at the 2-position by hydroxy, halogen, alkoxy, amino or substituted amino substituents and on the pyrimidine ring by alkyl substituents, or alkyl and halogen- substituted alkyl substituents.

W. Broadbent et al., *J. Chem. Soc., 1965,* 3369, discloses the compound 3-mercapto-7-methyl-5-n-propyl-1,2,4-triazolo[4,3-c]pyrimidine. This publication also discloses 1,2,4-triazolo[1,5-c]pyrimidines (referred to therein as triazolo[2,3-c]pyrimidines) which are substituted at the 2-position by a mercapto, alkylthio, alkylsulphonyl, or dialkylamino substituent, and on the pyrimidine ring by alkyl substituents or alkyl and halogen-substituted alkyl substituents.

Still other 1,2,4-triazolo[4,3-c]pyrimidines and 1,2,4-triazolo[1,5-c]pyrimidines are disclosed in the following articles and patents.

Shiho et al., *Yakagaku Zasshi,* 1956, *76,* 804, discloses 1,2,4-triazolo[4,3-c]pyrimidines which are substituted at the 3-position by alkyl or phenyl substituents, and on the pyrimidine ring by both methyl and methoxy substituents.

Temple et al., *J. Org. Chem.,* 1968, *33,* 530, discloses the compounds 8-amino-7-chloro-s-triazolo[4,3-c]-pyrimidine-3(2H)-one and 8-amino-7-chloro-s-triazolo[1,5-c]pyrimidine-2(3H)-one.

D. J. Brown et al., *Aust. J. Chem.,* 1978, *31,* 2505, discloses 1,2,4-triazolo[4,3-c]pyrimidines which are substituted at the 3-position by hydrogen or alkyl substituents, and on the pyrimidine ring by hydrogen and/or alkyl substituents. This publication also discloses 1,2,4-triazolo[1,5-c]pyrimidines which are substituted at the 2-position by hydrogen or an alkyl substituent, and on the pyrimidine ring by hydrogen and/or alkyl substituents.

D. J. Brown et al., *Aust. J. Chem.,* 1979, *32,* 1585, discloses 1,2,4-triazolo[4,3-c]pyrimidines which are

substituted at the 3-position by hydrogen or an alkyl substituent, and on the pyrimidine ring at the 5-position by a halogen, hydrazino, methylthio or methyl substituent, and at the 7-position by a methyl substituent. This publication also discloses 1,2,4-triazolo[1,5-c]pyrimidines which are substituted at the 2-position by hydrogen or an alkyl substituent, and on the pyrimidine ring at the 5-position by a halogen, hydrazino, alkyl or alkylthio substituent, and at the 7-position by an alkyl substituent.

D. J. Brown et al., *Aust. J. Chem.*, 1980, *33*, 1147, discloses pyrimidines which are substituted at the 2-position by hydrogen or an alkyl or phenyl substituent, and on the pyrimidine ring at the 5-position by halogen, dimethylaminoethyleneamino, hydroxyaminomethyleneamino or 5-acetoxyaminomethyleneamino, and at the 7-position by hydrogen or an alkyl substituent.

U.S. Patent No. 4,269,980 discloses 5-, 7- and 8-(optionally substituted-phenyl)-1,2,4-triazolo[4,3-c]-pyrimidines which may be substituted at the 3-position by hydrogen or an alkyl substituent. This patent also discloses 5-, 7- and 8-(optionally substituted phenyl)-1,2,4-triazolo[1,5-c]pyrimidines which may be substituted at the 2-position by hydrogen or an alkyl substituent. These compounds are all anxiolytic agents.

FR—A—1,205,144 purports to disclose triazolo [4,3-c]pyrimidines of formula:

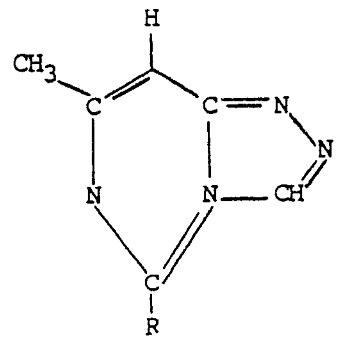

wherein R is NH, a primary, secondary, aliphatic, aromatic or heterocyclic amine, SH, $NHNH_2$ or alkoxy. These compounds are stated to be useful for lowering blood pressure.

However the compounds actually produced by the specified Examples are triazolo*[1,5-c]*pyrimidines.

This document also discloses a process for the production of triazolo pyrimidines via the intermediate 5-chloro-7-methyl-S.triazol pyrimidine, which is obtained by reacting 5-hydroxy-7-methyl-S.triazol pyrimidine with phosphrus oxychloride in the presence of N.N.dimethylaniline. These compounds are purported to be the [4,3-c] structural isomers but are in fact the [1,5-c] isomers.

Detailed Description of the Invention

The present invention relates to 1,2,4-triazolo[4,3-c]pyrimidines and 1,2,4-triazolo[1,5-c]pyrimidines which are bronchodilators. The invention also relates to pharmaceutical compositions comprising an effective amount of a 1,2,4-triazolo[4,3-c]pyrimidine or 1,2,4-triazolo[1,5-c]pyrimidine of the invention and a pharmaceutically acceptable carrier.

Specifically, the present invention relates to compounds of the formula IA

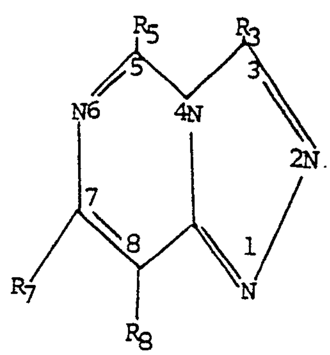

wherein $R_3$ is hydrogen or alkyl of 1 to 4 carbon atoms; one of $R_5$ and $R_7$ is the heterocyclic substituent

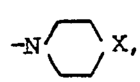

wherein X is oxygen, sulfur, sulfinyl, sulfonyl, methylene, imino or n-alkylamino of 1 to 4 carbon atoms; when $R_5$ is said heterocyclic substituent, $R_7$ is hydrogen or alkyl of 1 to 4 carbon atoms; when $R_7$ is said

heterocyclic substituent, $R_5$ is hydrogen, alkyl or alkylthio of 1 to 4 carbon atoms, or phenyl; and $R_8$ is hydrogen, phenyl or alkyl of 1 to 4 carbon atoms; or a pharmaceutically acceptable salt thereof.

The present invention also relates to compounds of the formula IB

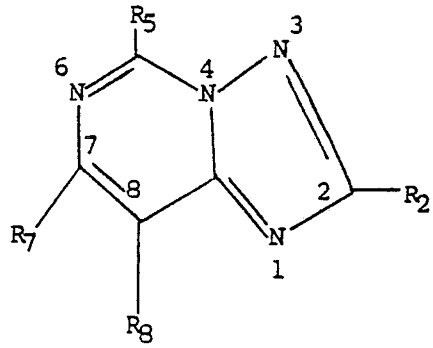

IB

wherein $R_2$ is hydrogen or alkyl of 1 to 4 carbon atoms; at least one of $R_5$ and $R_7$ is

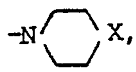

wherein the or each X is independently oxygen, sulphur, sulphinyl, sulphonyl, methylene, imino or N-alkylimino of 1 to 4 carbon atoms, the other of $R_5$ and $R_7$, if not

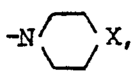

being hydrogen, alkyl of 1 to 4 carbon atoms, or phenyl; and $R_8$ is hydrogen, alkyl of 1 to 4 carbon atoms or phenyl; with the proviso that if $R_5$ is

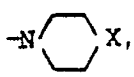

and $R_7$ is methyl, then X is sulphinyl or sulphonyl.

In accordance with the invention, compounds for pharmaceutically acceptable salts thereof both as defined in formula IA and IB, and also as defined in formula IB but without said proviso, may be used for the manufacture of a medicament for inducing bronchodilation in a mammal.

"Lower alkyl" as used in the instant specification designates straight or branched-chain alkyl groups containing one to four carbon atoms. Preferred lower alkyl substituents are methyl and ethyl.

The presently preferred compounds of Formulas IA and IB are those wherein X is sulfur or oxygen. Preferably $R_8$ is hydrogen. These compounds are preferred because of their generally higher potency in protecting against histamine-induced contraction of isolated guinea pig tracheal tissue. This assay is discussed in greater detail below.

Compounds of Formula IB are preferred because of their generally higher potency in protecting against histamine-induced contraction of isolated guinea pig tracheal tissue.

Specific examples of preferred compounds of Formula IA which are active in the aforementioned assay at concentrations of 10 µg per ml or lower are:

3,5-bis(n-propyl)-7-(4-thiomorpholino)-1,2,4-triazolo[4,3-c]pyrimidine
5-ethyl-3-methyl-7-(4-thiomorpholino)-1,2,4-triazolo[4,3-c]pyrimidine
3-methyl-5-methylthio-8-phenyl-7-(4-thiomorpholino)-1,2,4-triazolo[4,3-c]pyrimidine
5-ethyl-3-isopropyl-7-(4-thiomorpholino)-1,2,4-triazolo[4,3-c]pyrimidine
3-ethyl-5-methyl-7-(4-thiomorpholino)-1,2,4-triazolo[4,3-c]pyrimidine
3,5-bis(n-propyl)-7-(4-morpholino)-1,2,4-triazolo[4,3-c]pyrimidine
3,5-diethyl-7-(1-piperidino)-1,2,4-triazolo[4,3-c]pyrimidine
3-isopropyl-7-(4-morpholino)-5-*n*-propyl-1,2,4-triazolo[4,3-c]pyrimidine
7-(4-morpholino)-5-methylthio-1,2,4-triazolo[4,3-c]pyrimidine
3-ethyl-5-*n*-propyl-7-(4-thiomorpholino)-1,2,4-triazolo[4,3-c]pyrimidine
7-(4-methyl-1-piperazino)-5-(n-propyl)-1,2,4-triazolo[4,3-c]pyrimidine
5-*n*-propyl-7-(4-thiomorpholino)-1,2,4-triazolo[4,3-c]pyrimidine
5-ethyl-7-(4-morpholino)-1,2,4-triazolo[4,3-c]pyrimidine
5-ethyl-3-methyl-7-(4-morpholino)-1,2,4-triazolo[4,3-c]pyrimidine
3,5-diethyl-7-(4-thiomorpholino)-1,2,4-triazolo[4,3-c]pyrimidine

Specific examples of preferred compounds of Formula IB which are active in the aforementioned assay at concentrations of 5 μg per ml or lower are:

2-methyl-5-(4-thiomorpholino)-1,2,4-triazolo[1,5-c]pyrimidine
2-ethyl-7-methyl-5-(4-thiomorpholino)-1,2,4-triazolo[1,5-c]pyrimidine
5,7-bis(4-morpholino)-1,2,4-triazolo[1,5-c]pyrimidine
5-(4-morpholino)-7-(4-thiomorpholino)-1,2,4-triazolo[1,5-c]pyrimidine
2-ethyl-5-methyl-7-(4-thiomorpholino)-1,2,4-triazolo[1,5-c]pyrimidine
2-ethyl-5-methyl-7-(4-morpholino)-1,2,4-triazolo[1,5-c]pyrimidine
2,5-diethyl-7-(4-thiomorpholino)-1,2,4-triazolo[1,5-c]pyrimidine
2-ethyl-7-(4-morpholino)-5-*n*-propyl-1,2,4-triazolo[1,5-c]pyrimidine
7-(4-morpholino)-5-*n*-propyl-1,2,4-triazolo[1,5-c]pyrimidine
2-ethyl-5-*n*-propyl-7-(4-thiomorpholino)-1,2,4-triazolo[1,5-c]pyrimidine
3-*n*-propyl-7-(4-thiomorpholino)-1,2,4-triazolo[1,5-c]pyrimidine
5-ethyl-7-(4-morpholino)-1,2,4-triazolo[1,5-c]pyrimidine
5-ethyl-2-methyl-7-(4-morpholino)-1,2,4-triazolo[1,5-c]pyrimidine
2,5-diethyl-7-(4-morpholino)-1,2,4-triazolo[1,5-c]pyrimidine
2,5-diethyl-7-[4-(1-dioxothiomorpholino)]-1,2,3-triazolo[1,5-c]pyrimidine
2,5-diethyl-7-[4-(1-oxothiomorpholino)]-1,2,4-triazolo[1,5-c]pyrimidine

The bronchodilator activity of the compounds of Formulas IA and IB was assessed by the measurement of effects on isolated tracheal spirals. This is a well-known and long established *in vitro* test method. The bronchodilator activity was determined according to the following procedure: Female guinea pigs were sacrificed and each trachea removed and cut into a spiral strip. This strip was mounted in a constant temperature (37°C) muscle bath having a volume of approximately 15 ml. The bathing medium was Krebs-Henseleit solution. Movement of the tracheal strip was measured by means of an isometric transducer connected to an electric recorder. The bath was aerated with a mixture of 95% carbon dioxide and 5% oxygen. Contractions were induced in the strips by the addition of a suitable amount of histamine, acetylcholine or barium chloride. The amount of a given compound of Formula I (measured in μg/ml) required to provide greater than 75% relaxation of drug-induced contraction is considered an effective concentration. For comparison, a well known standard bronchodilator, aminophylline, requires concentrations of 50 μg/ml versus histamine, 100 μg/ml versus acetylcholine and 10 μg/ml versus barium chloride to provide greater than 75% relaxation.

The compounds of Formulas IA and IB which were most active in the *in vitro* test, including most of those listed above as preferred compounds, were tested *in vivo* in the guinea pig for oral activity in the so-called histamine aerosol method described in U.S. Patent 3,248,292. This test was modified slightly in that a 0.1% aqueous solution of histamine was used as the agent for inducing bronchial constriction. Oral doses were measured in mg/kg of body weight of the guinea pig.

Some of the compounds of Formulas IA and IB were also found to have activity as mucolytics in an *in vitro* test for mucus production in which rats are orally dosed with compound prior to sacrifice. The trachea is then isolated and incubated with radiolabelled glucosamine and the effect of compounds on the incorporation of glucosamine into extracellular mucus is determined. An active compound reduces incorporation of glucosamine. Specific examples of preferred compounds which are active in this assay are:

5-Methyl-7-thiomorpholino-1,2,4-triazolo[4,3-c]pyrimidine
7-Methyl-5-(4-morpholino)-1,2,4-triazolo[1,5-c]pyrimidine
2,7-Dimethyl-5-(4-methyl-1-piperazinyl)-1,2,4-triazolo[1,5-c]pyrimidine
2,7-Dimethyl-5-(1-piperazinyl)-1,2,4-triazolo[1,5-c]pyrimidine.

The compounds of Formulas IA and IB may be administered to mammals in order to induce bronchodilation. The compounds may be administered orally, parenterally or by inhalation. Preferably they are administered orally in tablets or capsules. The usual effective human dose will be in the range of 0.1 to 50 mg/kg of body weight.

Salts of compounds of formulas IA and IB are generally prepared by reaction with an equimolar amount of a relatively strong acid, preferably an inorganc acid such as hydrochloric, sulfuric or phosphoric acid, in a polar solvent. Isolation of the salt is facilitated by the addition of a solvent in which the salt is insoluble, an example of such a solvent is diethyl ether.

The compounds of Formulas IA and IB, either as the free base or in the form of a pharmaceutically acceptable acid-addition salt, can be combined with conventional pharmaceutical diluents and carriers to form such dosage forms as tablets, capsules, suspensions, solutions, suppositories and the like.

The pharmaceutical carrier employed may be, for example, either a solid or liquid. Examples of solid carriers are lactose, terra alba, sucrose, talc, gelatin, agar, pectin, acacia, magnesium stearate, stearic acid, and the like. Liquid carriers include syrup, peanut oil, olive oil, water and the like. Similarly, the carrier or diluent can include a time delay material well known to the art, such as glyceryl monostearate or glyceryl distearate, these being employed alone or, for example, in combination with a wax.

The compounds of Formulas IA and IB may be prepared by several synthetic routes. One such route is that shown in Scheme I below. This route is useful in preparing compounds wherein $R_5$ is hydrogen, lower alkyl or phenyl; $R_7$ is

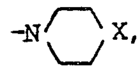

and $R_2$, $R_8$ and X are as defined previously. Each "alk" appearing in Scheme I is independently lower alkyl.

Scheme I

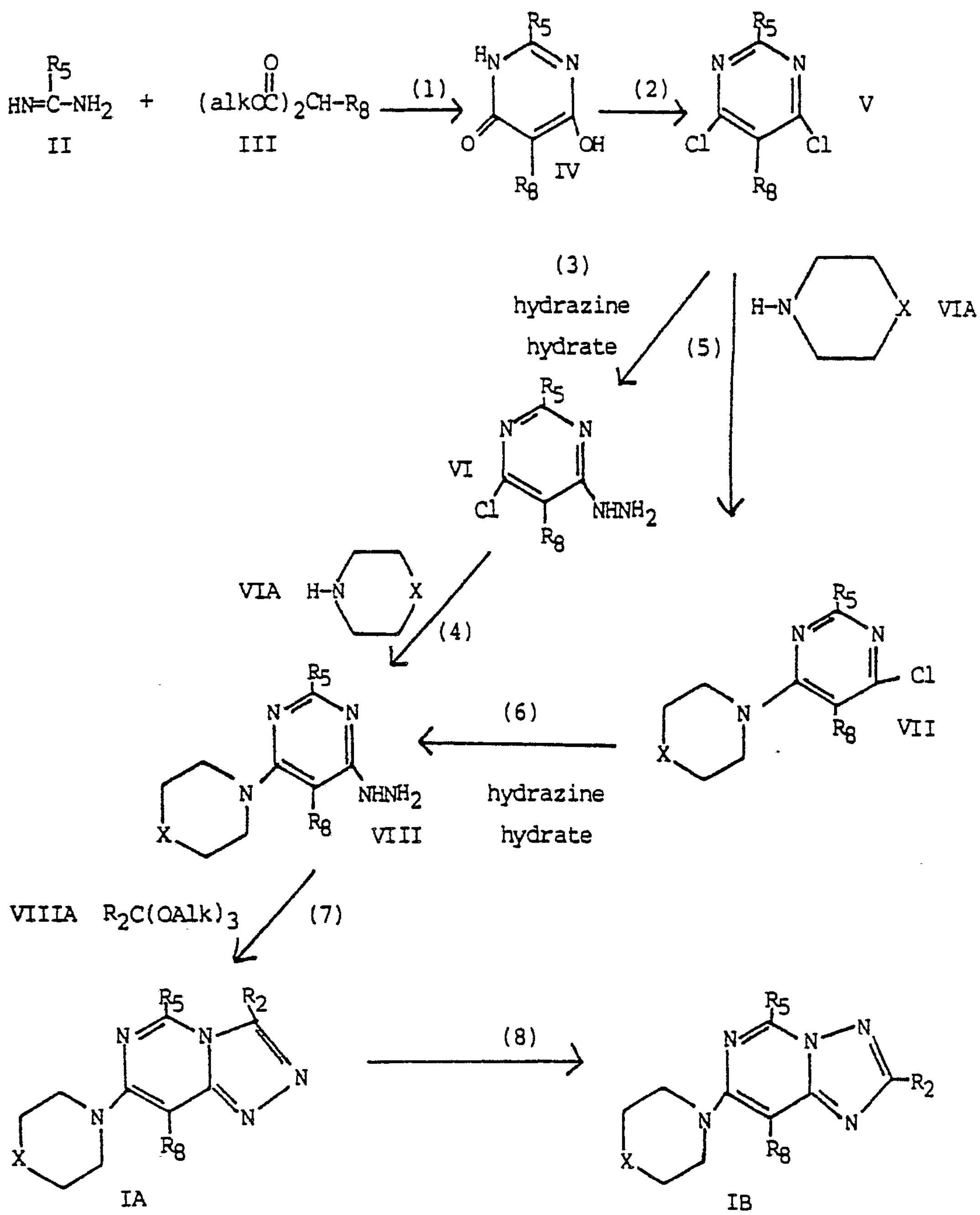


The reactions of steps (1), (2) and (3) have previously been reported for the preparation of compounds wherein $R_5$ is hydrogen, methyl or ethyl and $R_8$ is hydrogen or methyl. Thus, most of the compounds of formulas IV, V, and VI are known. Heterocyclic compounds of formulas VII, VIII and IX are novel. The known methods were used to carry out the reactions of steps (1), (2) and (3). Specifically, steps (1) and (2) were carried out as described in H. R. Henze et al., *J. Org. Chem.,* 1952, *17,* 1320 and H. R. Henze et al., *J. Org. Chem.,* 1953, *18,* 653, and step (3) was carried out as described in J. Chesterfield et al., *J. Chem. Soc.,* 1955, 3478.

Step (4) is carried out by reacting the optionally substituted 4-chloro-6-hydrazinopyrimidine of Formula VI with a heterocyclic amine of the formula VIA. The reactants are heated together without solvent or optionally (and preferably) in any solvent which does not participate in the reaction such as water. Two equivalents of the heterocyclic amine are preferably used. Alternatively, one equivalent of the heterocyclic amine may be replaced by an inorganic base to neutralize the hydrogen chloride, but lower yields are obtained. The reaction mixture is heated at a temperature up to or at its reflux temperature. A temperature is chosen which provides an adequate reaction rate. When water is used as the solvent, the temperature is generally in the range of 80 to 110°C. Good yields of the desired products are isolated by conventional methods such as filtration, extraction or chromatography. The novel intermediates of Formula VIII, which may also be prepared alternatively by following steps (5) and (6), are solids whose structural assignments are confirmed by infrared and nuclear magnetic resonance spectral analyses.

Step (5) is carried out by reacting optionally substituted 4,6-dichloropyrimidines of Formula V with heterocyclic amines of the formula VIA. This reaction is carried out by heating the reactants without solvent, or preferably in any solvent which does not participate in the reaction. Two equivalents of the heterocyclic amine are preferably used, one to react with the chloropyrimidine and the other to neutralize the hydrogen chloride by-product. Alternatively, an inorganic base may be used to neutralize the hydrogen chloride by-product, but lower yields of the desired product are generally obtained. Heating is at a temperature up to and including the refluxed temperature of the mixture. A temperature is chosen which provides an adequate reaction rate. If water is used as a solvent, the mixture is generally heated at its reflux temperature. Good yields of the desired product are isolated by conventional methods such as filtration, extraction or chromatography. The novel intermediates of Formula VII are solids. Structural assignments are confirmed by infrared and nuclear magnetic resonance spectral analyses.

Step (6) is carried out by reacting the novel substituted 4-chloro-6-heterocyclicaminopyrimidine of Formula VII wih hydrazine hydrate. The reaction is facile and is generally carried out at moderate temperatures, for example, from −20°C to the reflux temperature of the solvent. The reaction is generally carried out by adding two equivalents of hydrazine hydrate to a solution of the pyrimidine. The solvent will generally be a lower alkanol. The product is separated by conventional methods such as filtration, extraction or chromatography and is the same novel intermediate of Formula VIII obtained from step (4).

Step (7) is carried out by reacting the intermediate of Formula VIII with an orthoester of formula VIIA. Such orthoesters are well known and readily available. Examples of suitable orthoesters include trimethyl orthoformate, triethyl orthoformate, triethyl orthoacetate, triethyl orthopropionate and the like. Since the orthoesters are liquids, it is convenient to mix the intermediates of Formula VIII with an excess of orthoester and to heat the mixture at reflux until reaction is complete. Good yields of the desired solid compounds of Formula IA are obtained by conventional methods.

In step (8), the 1,2,4-triazolo[4,3-c]pyrimidines of Formula IA are heated with an aqueous acid and thereby converted to the compounds of Formula IB wherein $R_7$ is

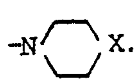

The preferred aqueous acids are carboxylic acids such as formic acid, acetic acid and propionic acid. The reaction mixture is generally heated at reflux for up to several days. The desired product is isolated by conventional methods. The structural assignments are made based on infrared and nuclear magnetic resonance spectral analyses. The products are generally white crystalline solids.

In some cases step (8) may be accomplished by continued heating of the reactants of step (7). This conversion occurs most readily when $R_5$ is hydrogen, and is carried out by using dimethyl sulfoxide as the solvent for the combined steps (7) and (8) as described in Example 104.

Synthetic Scheme II shows a method for the preparation of compounds of Formulas IA and IB wherein both $R_5$ and $R_7$ are

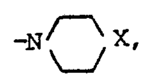

wherein X is as defined previously and may be the same or different in the two heterocyclic amino groups; and $R_2$ and $R_8$ are as defined previously. Alk is as defined previously.

Scheme II

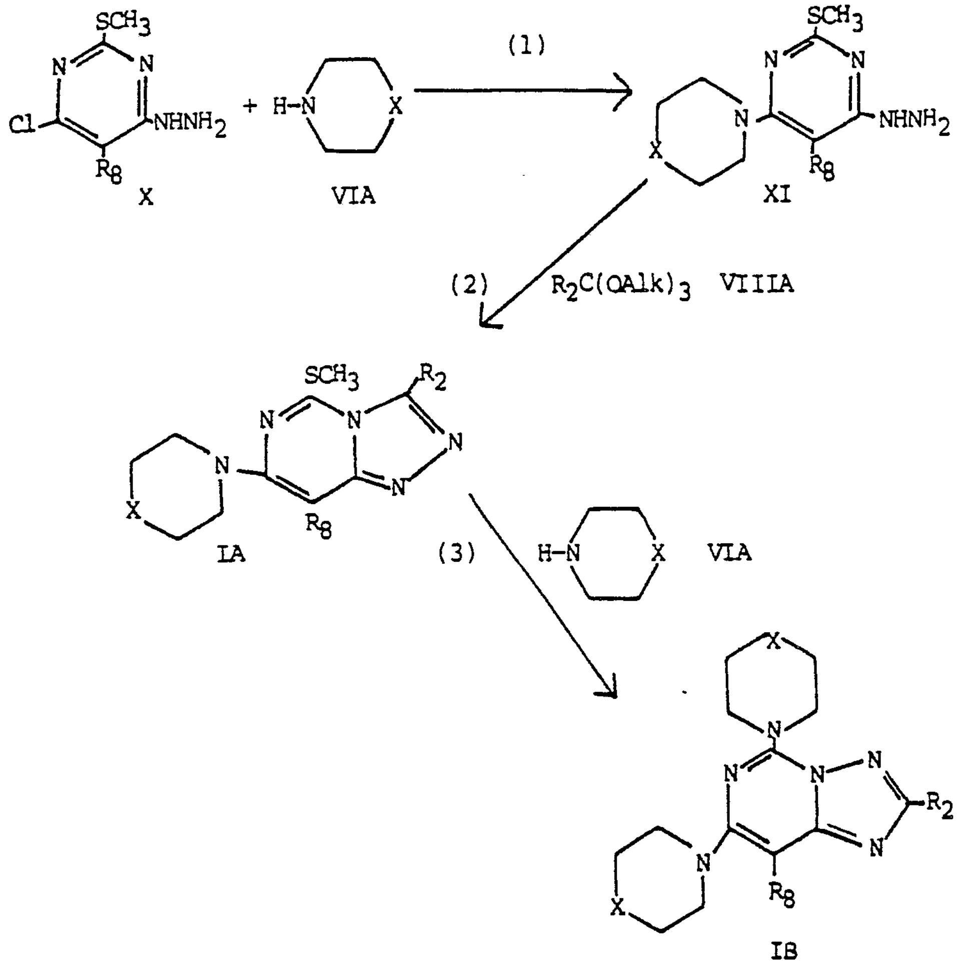


Step (1) of Scheme II requires reaction of the 4-chloro-6-hydrazino-2-methylthiopyrimidine of Formula X with a heterocyclic amine of the Formula VIA. Generally the 4-chloro-6-hydrazino-2-methylthiopyrimidines of Formula X are known compounds or may be prepared by conventional methods. The reaction is generally conducted in an inert solvent, preferably water, optionally in the presence of an acid acceptor such as a tertiary organic amine, for example, triethylamine. The reaction is best carried out using two equivalents of the amine of Formula VIA, one to react with the 4-chloro-6-hydrazino-2-methylthiopyrimidine and one to react with the hydrochloric acid which results. The mixture is heated at reflux for several hours, then cooled. Usually the novel intermediate of Formula XI is obtained directly as a solid precipitate. Alternatively, it is obtained by extraction or chromatographic techniques.

Step (2) of Scheme II is carried out by mixing the intermediate of Formula XI with an orthoester of Formula VIIIA. The reaction is carried out as described for step (7) of Scheme I. The product obtained is a compound of Formula IA. The product of this step generally is a mixture which is separated by chromatography, preferably high pressure liquid chromatography, to provide the crystalline solid. Occasionally the desired isomer is obtained in such high purity that chromatographic separation is unnecessary.

Step (3) of Scheme II requires heating of the compound of Formula IA in an excess of the heterocyclic amine of the Formula VIA, optionally in an inert solvent such as diglyme or dioxane. The reaction is generally carried out at the reflux temperature of the mixture. The product is isolated by conventional methods such as filtration, extraction or chromatography.

An alternative scheme, used for preparing compounds of Formula IB wherein $R_5$ is

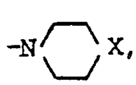

wherein X is as defined previously; $R_7$ is hydrogen or lower alkyl; and $R_2$ and $R_8$ are as defined previously, is shown in Scheme III.

Scheme III

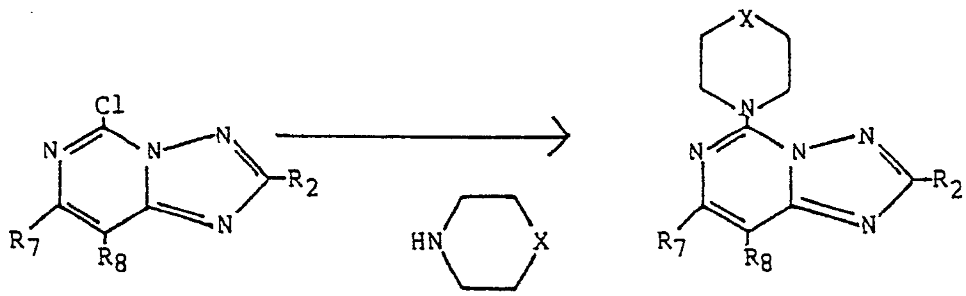

XIV VIA IB

The compounds of Formula XIV are generally known or may be prepared by conventional methods. Known methods may be employed to vary substituents $R_2$ and $R_7$. The reaction of Scheme III occurs readily at moderate temperatures, for example from 0°C up to the reflux temperature of the solvent. It is carried out either by adding the heterocyclic amine of Formula VIA to a solution of the intermediate of Formula XIV or vice versa. The solvent may be inert solvent, for example water or dioxane. The product of Formula IB prepared in Scheme III is obtained in good yields by conventional isolation methods.

Synthetic Scheme IV illustrated below is a method for preparation of compounds of Formulas IA and IB wherein $R_2$ and $R_7$ are independently hydrogen or lower alkyl; $R_5$ is

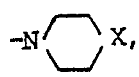

wherein X is as defined previously; and $R_8$ is as defined previously.

Scheme IV

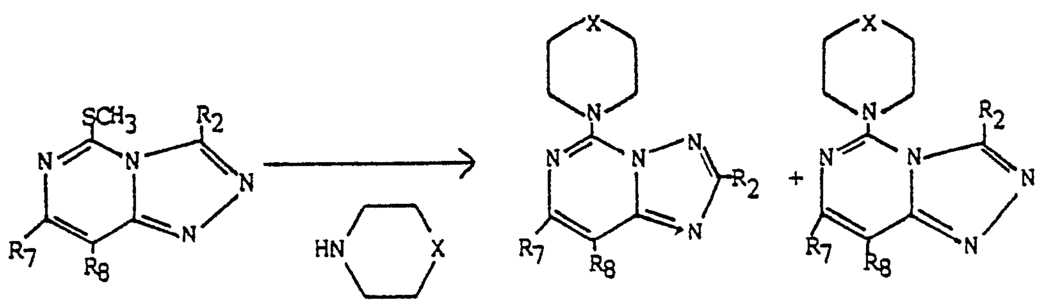

XV VIA IB IA

The reaction of Scheme IV is similar to reaction of step (3) of Scheme II. The intermediates of Formula XV are known or may be prepared from known starting materials using known methods. The products of Scheme IV are prepared and isolated as described for that step.

The following examples are provided to illustrate the methods used in the invention. They are not intended to limit the invention.

Example 1

Preparation of 3,5-Diethyl-7-(4)methyl-1-piperazino)-1,2,4-triazolo[4,3-c]pyrimidine and 2,7-Diethyl-5-(4-methyl-1-piperazinyl)-1,2,4-triazolo[1,5-c]pyrimidine according to Scheme I.

Part A. Preparation of 2-Ethyl-6-hydrazinyl-4-(4-methyl-1-piperazinyl)pyrimidine according to Step (4)

A mixture of 8.3 g (0.05 mole) of 4-chloro-2-ethyl-6-hydrazinylpyrimidine and 11 g (0.10 mole) of 1-methylpiperazine in 250 ml of water was heated at reflux for 16 hours and was then cooled and extracted with chloroform. The extracts were dried over magnesium sulfate and were then evaporated to provide 8.3 g (70%) of 2-ethyl-6-hydrazinyl-4-(4-methyl-1-piperazinyl)pyrimidine.

Part B. Preparation of 3,5-Diethyl-7-(4-methyl-1-piperazinyl)-1,2,4-triazolo[4,3-c]pyrimidine according to Step (7)

A mixture of 8.3 g (0.045 mole) of 2-ethyl-6-hydrazinyl-4-(4-methyl-1-piperazinyl)pyrimidine and 75 ml of triethyl orthopropionate was heated at reflux for 48 hours. After cooling the mixture was evaporated *in*

*vacuo*. Diethyl ether was added to the residue and the mixture was cooled. The precipitate was collected by filtration to provide 4.5 g (37%) of 3,5-diethyl-7-(4-methyl-1-piperazinyl)-1,2,4-triazolo[4,3-c]pyrimidine. Recrystallization from ethyl acetate-hexane and then from ethyl acetate-cyclohexane provided a white crystalline product, m.p. 128—131°C. Analysis for $C_{14}H_{22}N_6$: Calculated: %C, 61.3; %H, 8.1: %N, 30.6; Found: %C, 59.8; %H, 8.2; %N, 30.1. The structural assignment was confirmed by nuclear magnetic resonance and infrared spectral analyses.

Part C. Preparation of 2,5-Diethyl-7-(4-methyl-1-piperazinyl)-1,2,4-triazolo[1,5-c]pyrimidine according to step (8)

A mixture of 3.3 g of 3,5-diethyl-7-(4-methyl-1-piperazinyl)-1,2,4-triazolo[4,3-c]pyrimidine and 50 ml of 97% formic acid was heated at reflux for 18 hours. The mixture was cooled and evaporated *in vacuo* to provide a residue which was diluted with 100 ml of water and carefully neutralized with sodium bicarbonate. The solution was extracted with chloroform. The extracts were dried and then concentrated to provide an oil which solidified and was collected by filtration, washed with water and dried. Recrystallization from hexane provided off-white crystalline solid 2,5-diethyl-7-(4-methyl-1-piperazinyl)-1,2,4-triazolo[1,5-c]pyrimidine, m.p. 87—88°C. Analysis: Calculated for $C_{14}H_{22}N_6$: %C, 61.3; %H, 8.1: %N, 30.6; Found: %C, 60.6; %H, 8.1; %N, 30.6. The structural assignment was confirmed by infrared and nuclear magnetic resonance spectral analyses.

Examples 2—21

Using the method of Part A, Example 1, the indicated intermediate amines of Formula VIA were reacted with the indicated known 4-chloro-2-alkyl-6-hydrazinylpyrimidines of Formula VI to provide the novel intermediates of Formula VIII (Table I).

TABLE I

| Example | Amine Intermediate | Pyrimidine Intermediate of Formula VI | Intermediate of Formula VIII | Melting Point (in°C) |
|---|---|---|---|---|
| 2 | 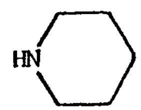 | 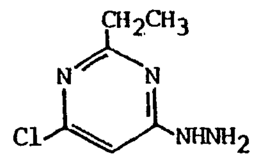 | 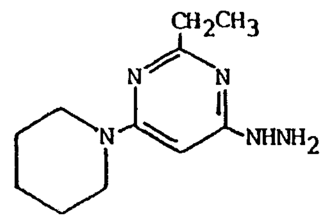 | none taken (yield 66%) |
| 3 | 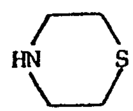 | " | 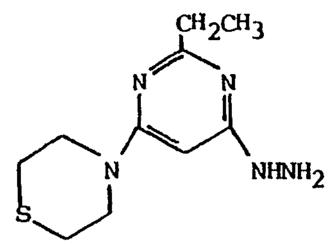 | 158-160 (yield 94.5%) |
| 4 | 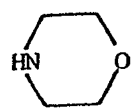 | " | 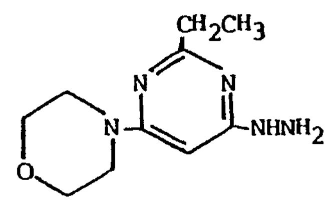 | 129-133 (yield 75%) |

Table I (continued)

| Example | Amine Intermediate | Pyrimidine Intermediate of Formula VI | Intermediate of Formula VIII | Melting Point (in°C) |
|---|---|---|---|---|
| 5 | 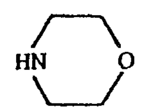 | 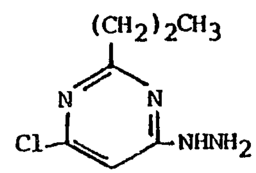 | 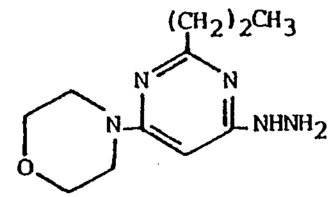 | 112-115 |
| 6 | 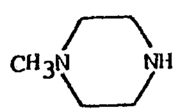 | 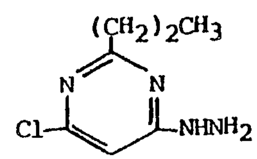 | 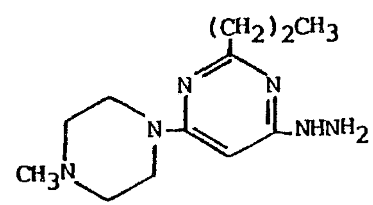 | none taken (yield 84%) |
| 7 | 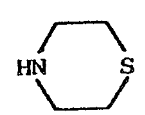 | 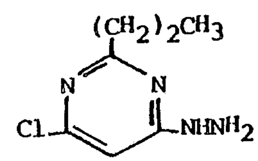 | 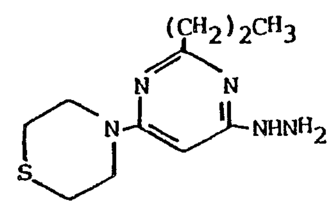 | 144-146 (yield 71%) |

Table I (Continued)

| Example | Amine Intermediate | Pyrimidine Intermediate of Formula VI | Intermediate of Formula VIII | Melting Point (in°C) |
| --- | --- | --- | --- | --- |
| 8 | 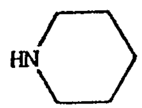 | 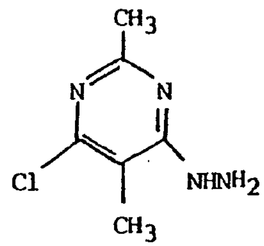 | 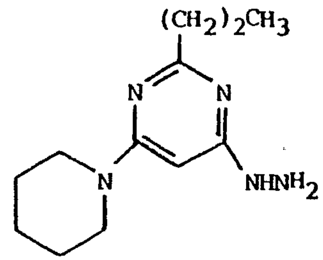 | none taken (yield 85%) |
| 9 | 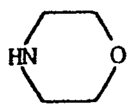 | 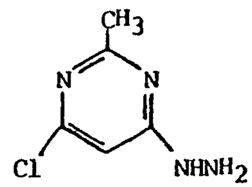 | 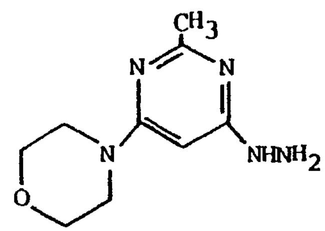 | none taken (yield 68%) |
| 10 | 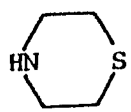 | 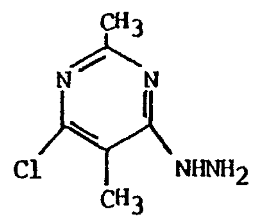 | 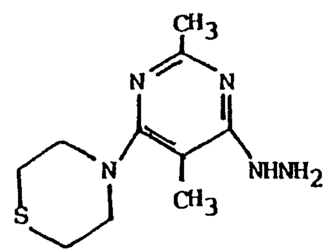 | 183-185 |

Table I (Continued)

| Example | Amine Intermediate | Pyrimidine Intermediate of Formula VI | Intermediate of Formula VIII | Melting Point (in°C) |
|---|---|---|---|---|
| 11 | 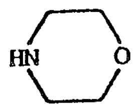 | 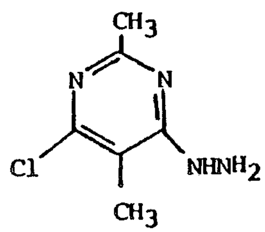 | 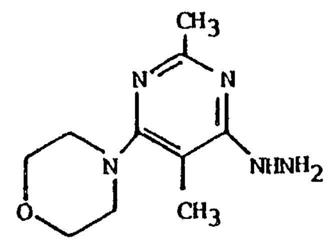 | none taken (yield 66%) |
| 12 | 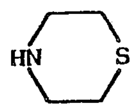 | 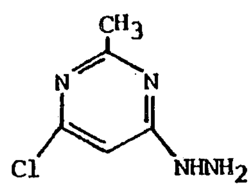 | 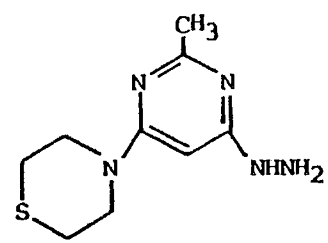 | 203-204 |
| 13 | 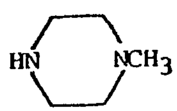 | 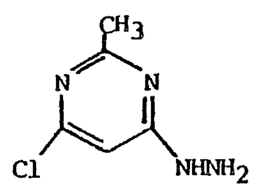 | 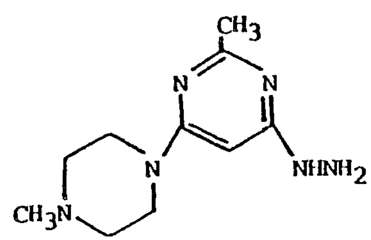 | 174-177 |

Table I (Continued)

| Example | Amine Intermediate | Pyrimidine Intermediate of Formula VI | Intermediate of Formula VIII | Melting Point (in°C) |
|---|---|---|---|---|
| 14 | 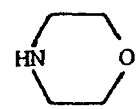 | 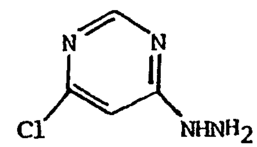 | 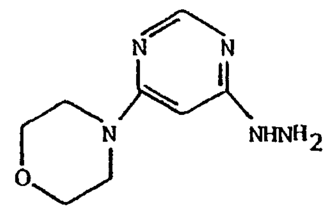 | 155-157 |
| 15 | 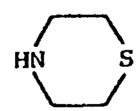 | 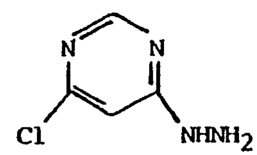 | 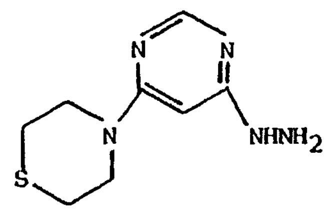 | 147-149 |
| 16 | 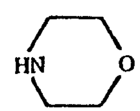 | 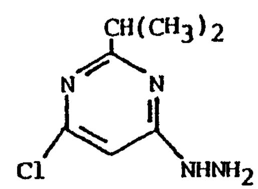 | 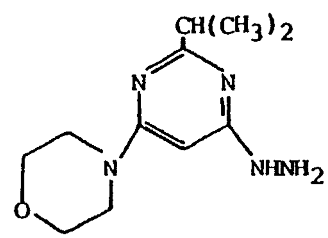 | 122-123 |

Table I (Continued)

| Example | Amine Intermediate | Pyrimidine Intermediate of Formula VI | Intermediate of Formula VIII | Melting Point (in°C) |
| --- | --- | --- | --- | --- |
| 17 | 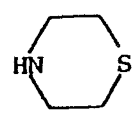 | 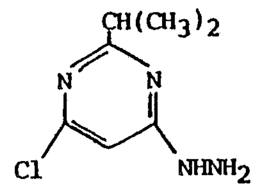 | 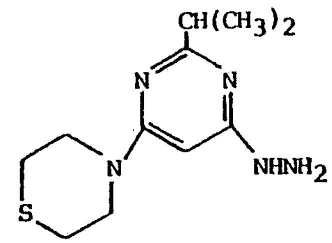 | 124-126 |
| 18 | 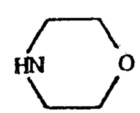 | 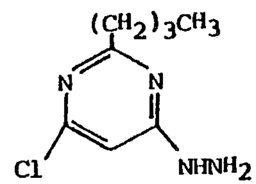 | 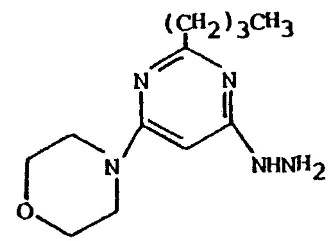 | none taken |
| 19 | 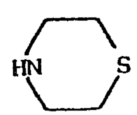 | 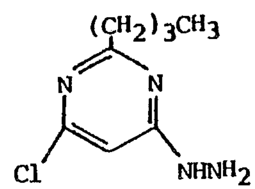 | 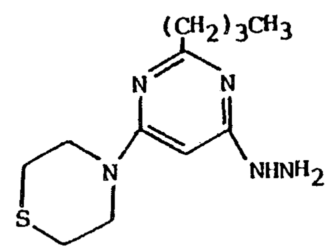 | none taken |

Table I (Continued)

| Example | Amine Intermediate | Pyrimidine Intermediate of Formula VI | Intermediate of Formula VIII | Melting Point (in°C) |
|---|---|---|---|---|
| 20 | 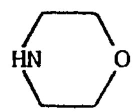 | 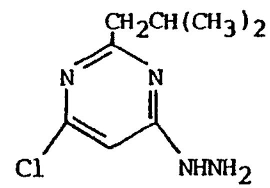 | 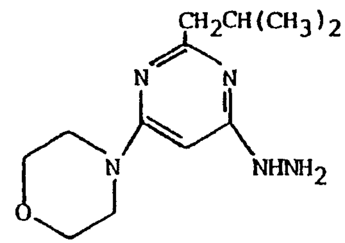 | (oil) |
| 21 | 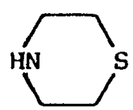 | 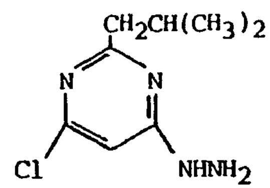 | 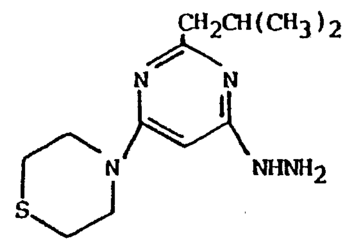 | (oil) |

Examples 22—59

Using the method of Part B, Example 1, the indicated intermediates of Formula VIII were reacted with the indicated trialkyl orthoesters to provide the novel compounds of Formula IA (Table II).

Table II

| Ex. | Intermediate of Formula VIII | | | Ortho Ester | Compound of Formula IA | | | | Calculated: %C; %H; %N<br>Found: %C; %H; %N<br>(m.p. in°C) |
|---|---|---|---|---|---|---|---|---|---|
| | $R_5$ | $R_8$ | X | | $R_3$ | $R_5$ | $R_8$ | X | |
| 22 | $CH_3$ | H | $SO_2$ | triethyl orthoformate | H | $CH_3$ | H | $SO_2$ | 44.9; 4.9; 26.2<br>44.9; 4.9; 26.3<br>(299-300) |
| 23 | $CH_3$ | H | $SO_2$ | triethyl orthoacetate | $CH_3$ | $CH_3$ | H | $SO_2$ | 47.0; 5.4; 25.0<br>47.1; 5.4; 24.9<br>(307-308) |
| 24 | $CH_2CH_2CH_3$ | H | O | triethyl orthoformate | H | $CH_2CH_2CH_3$ | H | O | 58.3; 6.9; 28.3<br>58.2; 7.0; 28.4<br>(200-202) |
| 25 | $CH_2CH_2CH_3$ | H | O | triethyl orthopropionate | $CH_2CH_3$ | $CH_2CH_2CH_3$ | H | O | 61.1; 7.7; 25.4<br>60.9; 7.9; 25.7<br>(179-181) |
| 26 | $CH_2CH_2CH_3$ | H | S | triethyl orthoformate | H | $CH_2CH_2CH_3$ | H | S | 54.7; 6.5; 26.6<br>54.6; 6.5; 26.9<br>(208-209) |

Table II (continued)

| Ex. | Intermediate of Formula VIII | | | Ortho Ester | Compound of Formula IA | | | | Calculated: %C; %H; %N<br>Found: %C; %H; %N<br>(m.p. in°C) |
|---|---|---|---|---|---|---|---|---|---|
| | $R_5$ | $R_8$ | X | | $R_3$ | $R_5$ | $R_8$ | X | |
| 27 | $CH_2CH_2CH_3$ | H | S | triethyl orthopropionate | $CH_2CH_3$ | $CH_2CH_2CH_3$ | H | S | 57.7; 7.3; 24.0<br>57.7; 7.5; 24.4<br>(143-144) |
| 28 | $CH_2CH_2CH_3$ | H | $CH_2$ | triethyl orthopropionate | $CH_2CH_3$ | $CH_2CH_2CH_3$ | H | $CH_2$ | (as $H_2SO_4$ salt):<br>48.5; 6.8; 18.9<br>48.4; 7.0; 19.0<br>(190-192) |
| 29 | $CH_3$ | H | S | triethyl orthoformate | H | $CH_3$ | H | S | 51.0; 5.6; 29.8<br>51.0; 5.5; 29.6<br>(234-237) |
| 30 | $CH_2CH_3$ | H | $CH_2$ | triethyl orthopropionate | $CH_2CH_3$ | $CH_2CH_3$ | H | $CH_2$ | 64.8; 8.2; 27.0<br>65.1; 8.3; 27.2<br>(120-122) |

Table II (continued)

| Ex. | Intermediate of Formula VIII | | | Ortho Ester | Compound of Formula IA | | | | Calculated: %C; %H; %N<br>Found: %C; %H; %N<br>(m.p. in°C) |
|---|---|---|---|---|---|---|---|---|---|
| | $R_5$ | $R_8$ | X | | $R_3$ | $R_5$ | $R_8$ | X | |
| 31 | $CH_2CH_3$ | H | S | triethyl orthopropionate | $CH_2CH_3$ | $CH_2CH_3$ | H | S | 56.3; 6.9; 25.2<br>56.1; 7.2; 25.5<br>(149–150) |
| 32 | $CH_2CH_3$ | H | S | triethyl orthoacetate | $CH_3$ | $CH_2CH_3$ | H | S | 54.7; 6.5; 26.6<br>54.4; 6.7; 26.7<br>(168–170) |
| 33 | $CH_2CH_3$ | H | S | triethyl orthoformate | H | $CH_2CH_3$ | H | S | 53.0; 6.0; 28.1<br>52.6; 6.2; 28.2<br>(243–245) |
| 34 | $CH_2CH_3$ | H | O | triethyl orthoacetate | $CH_3$ | $CH_2CH_3$ | H | O | 58.3; 6.9; 28.3<br>58.2; 7.0; 28.5<br>(200–203) |

Table II (continued)

| Ex. | Intermediate of Formula VIII | | | Ortho Ester | Compound of Formula IA | | | | Calculated: %C; %H; %N<br>Found: %C; %H; %N<br>(m.p. in°C) |
|---|---|---|---|---|---|---|---|---|---|
| | $R_5$ | $R_8$ | X | | $R_3$ | $R_5$ | $R_8$ | X | |
| 35 | $CH_2CH_3$ | H | O | triethyl orthopropionate | $CH_2CH_3$ | $CH_2CH_3$ | H | O | 59.7; 7.3; 26.8<br>59.6; 7.4; 27.0<br>(173–174) |
| 36 | $CH_2CH_3$ | H | O | triethyl orthoformate | H | $CH_2CH_3$ | H | O | 56.6; 6.5; 30.0<br>56.6; 6.4; 30.3<br>(223–224) |
| 37 | $CH_3$ | H | O | trimethyl orthoformate | H | $CH_3$ | H | O | 54.8; 6.0; 31.9<br>54.7; 6.0; 31.4<br>(208–210) |
| 38 | $CH_3$ | H | O | triethyl orthoacetate | $CH_3$ | $CH_3$ | H | O | 56.6; 6.5; 30.0<br>56.7; 6.3; 30.3<br>(171–173) |

Table II (continued)

| Ex. | Intermediate of Formula VIII | | | Ortho Ester | Compound of Formula IA | | | | Calculated: %C; %H; %N<br>Found: %C; %H; %N<br>(m.p. in°C) |
|---|---|---|---|---|---|---|---|---|---|
| | $R_5$ | $R_8$ | X | | $R_3$ | $R_5$ | $R_8$ | X | |
| 39 | $CH_3$ | H | O | triethyl orthopropionate | $CH_2CH_3$ | $CH_3$ | H | O | 58.3; 6.9; 28.3<br>57.9; 7.0; 28.3<br>(169-172) |
| 40 | $CH_3$ | H | S | triethyl orthoacetate | $CH_3$ | $CH_3$ | H | S | 53.0; 6.0; 28.1<br>53.1; 6.3; 28.3<br>(223-225) |
| 41 | $CH_3$ | H | S | triethyl orthopropionate | $CH_2CH_3$ | $CH_3$ | H | S | 54.8; 6.5; 26.6<br>54.4; 6.7; 26.8<br>(161-163) |
| 42 | $CH_3$ | $CH_3$ | S | triethyl orthoformate | H | $CH_3$ | $CH_3$ | S | 53.0; 6.0; 28.1<br>53.2; 6.3; 28.4<br>(182-184) |

Table II (continued)

| Ex. | Intermediate of Formula VIII $R_5$ | $R_8$ | X | Ortho Ester | Compound of Formula IA $R_3$ | $R_5$ | $R_8$ | X | Calculated: %C; %H; %N<br>Found: %C; %H; %N<br>(m.p. in°C) |
|---|---|---|---|---|---|---|---|---|---|
| 43 | $CH_3$ | $CH_3$ | S | triethyl orthoacetate | $CH_3$ | $CH_3$ | $CH_3$ | S | 54.7; 6.5; 26.6<br>54.4; 6.6; 26.9<br>(184-185) |
| 44 | $CH_3$ | $CH_3$ | S | triethyl orthopropionate | $CH_2CH_3$ | $CH_3$ | $CH_3$ | S | 56.3; 6.9; 25.2<br>56.4; 7.1; 25.1<br>(129-130) |
| 45 | $CH_3$ | $CH_3$ | $SO_2$ | triethyl orthoformate | H | $CH_3$ | $CH_3$ | $SO_2$ | 46.9; 5.4; 24.9<br>47.0; 5.3; 25.2<br>(234-236) |
| 46 | $CH_2CH_3$ | H | S | trimethyl orthobutyrate | $CH_2CH_2CH_3$ | $CH_2CH_3$ | H | S | 57.7; 7.3; 24.0<br>57.6; 7.5; 24.2<br>(163-165) |

Table II (continued)

| Ex. | Intermediate of Formula VIII | | | Ortho Ester | Compound of Formula IA | | | | Calculated: %C; %H; %N<br>Found: %C; %H; %N<br>(m.p. in°C) |
|---|---|---|---|---|---|---|---|---|---|
| | $R_5$ | $R_8$ | X | | $R_3$ | $R_5$ | $R_8$ | X | |
| 47 | $CH_2CH_3$ | H | O | trimethyl orthobutyrate | $CH_2CH_2CH_3$ | $CH_2CH_3$ | H | O | 61.1; 7.7; 25.4<br>61.0; 7.9; 25.8<br>(164-166) |
| 48 | $CH_2CH_3$ | H | O | trimethyl orthoisobutyrate | $CH(CH_3)_2$ | $CH_2CH_3$ | H | O | 61.1; 7.7; 25.4<br>61.0; 7.7; 25.3<br>(136-137) |
| 49 | $CH_2CH_3$ | H | S | trimethyl orthoisobutyrate | $CH(CH_3)_2$ | $CH_2CH_3$ | H | S | 57.7; 7.3; 24.0<br>57.8; 7.3; 24.2<br>(143-144) |
| 50 | $CH_2CH_2CH_3$ | H | S | trimethyl orthobutyrate | $CH_2CH_2CH_3$ | $CH_2CH_2CH_3$ | H | S | 59.0; 7.6; 22.9<br>59.0; 7.5; 23.1<br>(134-135) |

Table II (continued)

| Ex. | Intermediate of Formula VIII | | | Ortho Ester | Compound of Formula IA | | | | Calculated: %C; %H; %N<br>Found: %C; %H; %N<br>(m.p. in°C) |
|---|---|---|---|---|---|---|---|---|---|
| | $R_5$ | $R_8$ | X | | $R_3$ | $R_5$ | $R_8$ | X | |
| 51 | $CH_2CH_2CH_3$ | H | O | trimethyl orthobutyrate | $CH_2CH_2CH_3$ | $CH_2CH_2CH_3$ | H | O | 62.3; 8.0; 24.2<br>62.2; 8.1; 24.1<br>(139-140) |
| 52 | $CH_2CH_2CH_3$ | H | S | trimethyl orthoisobutyrate | $CH(CH_3)_2$ | $CH_2CH_2CH_3$ | H | S | 59.0; 7.6; 22.9<br>59.1; 7.7; 23.0<br>(134-136) |
| 53 | $CH_2CH_2CH_3$ | H | O | trimethyl orthoisobutyrate | $CH(CH_3)_2$ | $CH_2CH_2CH_3$ | H | O | 62.3; 8.0; 24.2<br>62.4; 8.1; 24.6<br>(116-117) |
| 54 | $CH(CH_3)_2$ | H | O | triethyl orthoformate | H | $CH(CH_3)_2$ | H | O | (as $H_2SO_4$ Salt):<br>41.7; 5.5; 20.3<br>41.6; 5.7; 20.6<br>(177-178) |

Table II (continued)

| Ex. | Intermediate of Formula VIII | | | Ortho Ester | Compound of Formula IA | | | | Calculated: %C; %H; %N<br>Found: %C; %H; %N<br>(m.p. in°C) |
|---|---|---|---|---|---|---|---|---|---|
| | $R_5$ | $R_8$ | X | | $R_3$ | $R_5$ | $R_8$ | X | |
| 55 | $CH(CH_3)_2$ | H | O | triethyl orthopropionate | $CH_2CH_3$ | $CH(CH_3)_2$ | H | O | (as $H_2SO_4$ Salt):<br>45.0; 6.2; 18.8<br>44.9; 6.3; 19.0<br>(167-168) |
| 56 | $CH(CH_3)_2$ | H | O | trimethyl orthobutyrate | $CH_2CH_2CH_3$ | $CH(CH_3)_2$ | H | O | (as $H_2SO_4$ Salt):<br>46.5; 6.5; 18.1<br>46.2; 6.5; 18.3<br>(162-163) |
| 57 | $CH(CH_3)_2$ | H | S | triethyl orthoacetate | $CH_3$ | $CH(CH_3)_2$ | H | S | (as $H_2SO_4$ Salt):<br>41.6; 5.6; 18.7<br>41.6; 5.8; 18.8<br>(170-172) |

Table II (continued)

| Ex. | Intermediate of Formula VIII | | | Ortho Ester | Compound of Formula IA | | | | Calculated: %C; %H; %N<br>Found: %C; %H; %N<br>(m.p. in°C) |
|---|---|---|---|---|---|---|---|---|---|
| | $R_5$ | $R_8$ | X | | $R_3$ | $R_5$ | $R_8$ | X | |
| 58 | $CH(CH_3)_2$ | H | S | triethyl orthopropionate | $CH_2CH_3$ | $CH(CH_3)_2$ | H | S | (as $H_2SO_4$ Salt):<br>43.2; 6.0; 18.0<br>43.1; 6.1; 18.2<br>(174-175) |
| 59 | $CH(CH_3)_2$ | H | O | triethyl orthoformate | H | $CH(CH_3)_2$ | H | O | (as $H_2SO_4$ Salt):<br>41.7; 5.5; 20.3<br>41.6; 5.7; 20.6<br>(177-178) |

Examples 60—96

Using the method of Part C, Example 1, the indicated compounds of Formula IA were heated with formic acid to provide the indicated compounds of Formula IB (Table III).

Table III

| Ex. No. | Compound of Formula IA; Product of Formula IB $R_2$ | $R_5$ | $R_8$ | X | Product of Formula IB Calculated: %C; %H; %N Found: %C; %H; %N |
|---|---|---|---|---|---|
| 60 | $CH_2CH_3$ | $CH_2CH_3$ | H | $CH_2$ | 64.8; 8.2; 27.0<br>65.1; 8.3; 27.0<br>m.p. 90-92°C |
| 61 | $CH_2CH_3$ | $CH_2CH_3$ | H | S | 56.3; 6.9; 25.2<br>56.1; 7.1; 25.0<br>m.p. 119-120°C |
| 62 | $CH_3$ | $CH_2CH_3$ | H | S | 54.7; 6.5; 26.6<br>54.8; 6.8; 26.5<br>m.p. 164-165°C |
| 63 | H | $CH_2CH_3$ | H | S | 53.0; 6.0; 28.1<br>52.9; 6.3; 28.4<br>m.p. 228-230°C |
| 64 | $CH_3$ | $CH_2CH_3$ | H | O | 58.3; 6.9; 28.3<br>58.3; 7.0; 28.3<br>m.p. 159-160°C |
| 65 | $CH_2CH_3$ | $CH_2CH_3$ | H | O | 59.7; 7.3; 26.8<br>59.7; 7.3; 26.9<br>m.p. 118-119°C |
| 66 | H | $CH_2CH_3$ | H | O | 56.6; 6.5; 30.0<br>56.8; 6.5; 30.1<br>m.p. 168-170°C |

Table III (continued)

| Ex. No. | Compound of Formula IA; Product of Formula IB: $R_2$ | $R_5$ | $R_8$ | X | Product of Formula IB Calculated: %C; %H; %N / Found: %C; %H; %N |
|---|---|---|---|---|---|
| 67 | H | $CH_3$ | H | $O_2S$ | 44.9; 4.9; 26.2<br>44.9; 4.9; 26.2<br>m.p. 251-253°C |
| 68 | $CH_3$ | $CH_3$ | H | $O_2S$ | 47.0; 5.4; 25.0<br>46.8; 5.4; 24.4<br>m.p. 229-233°C |
| 69 | H | $(CH_2)_2CH_3$ | H | O | 58.3; 6.9; 28.3<br>58.5; 7.0; 28.6<br>m.p. 153-155°C |
| 70 | $CH_2CH_3$ | $(CH_2)_2CH_3$ | H | O | 61.1; 7.7; 25.4<br>61.2; 7.7; 25.6<br>m.p. 130-131°C |
| 71 | H | $(CH_2)_2CH_3$ | H | S | 54.7; 6.5; 26.6<br>54.9; 6.5; 26.9<br>m.p. 145-150°C |
| 72 | $CH_2CH_3$ | $(CH_2)_2CH_3$ | H | S | 57.7; 7.3; 24.0<br>57.6; 7.3; 24.2<br>m.p. 101-103°C |
| 73 | $CH_2CH_3$ | $(CH_2)_2CH_3$ | H | $CH_2$ | 65.9; 8.5; 25.6<br>65.8; 8.6; 25.8<br>m.p. 60-62°C |

Table III (continued)

| Ex. No. | Compound of Formula IA; Product of Formula IB: $R_2$ | $R_5$ | $R_8$ | X | Product of Formula IB Calculated: %C; %H; %N Found: %C; %H; %N |
|---|---|---|---|---|---|
| 74 | H | $CH_3$ | H | S | 51.0; 5.5; 29.8<br>51.0; 5.7; 30.2<br>m.p. 154-156°C |
| 75 | H | $CH_3$ | H | O | 54.8; 6.0; 31.9<br>54.7; 5.8; 32.4<br>m.p. 159-161.5°C |
| 76 | $CH_3$ | $CH_3$ | H | O | 56.5; 6.5; 30.0<br>56.7; 6.6; 30.6<br>m.p. 182-183°C |
| 77 | $CH_2CH_3$ | $CH_3$ | H | O | 58.3; 6.9; 28.3<br>58.3; 7.0; 28.7<br>m.p. 146-147°C |
| 78 | $CH_3$ | $CH_3$ | H | S | 53.0; 6.0; 28.1<br>52.7; 6.1; 28.0<br>m.p. 170-172°C |
| 79 | $CH_2CH_3$ | $CH_3$ | H | S | 54.7; 6.5; 26.7<br>54.6; 6.7; 27.1<br>m.p. 124-125°C |
| 80 | H | $CH_3$ | $CH_3$ | S | 53.0; 6.0; 28.1<br>52.9; 6.2; 28.4<br>m.p. 150-152°C |

Table III (continued)

| Ex. No. | Compound of Formula IA; Product of Formula IB: $R_2$ | $R_5$ | $R_8$ | X | Product of Formula IB Calculated: %C; %H; %N Found: %C; %H; %N |
|---|---|---|---|---|---|
| 81 | $CH_3$ | $CH_3$ | $CH_3$ | S | 54.7; 6.5; 26.2<br>54.8; 6.5; 26.6<br>m.p. 128-130°C |
| 82 | $CH_2CH_3$ | $CH_3$ | $CH_3$ | S | 56.3; 6.9; 25.2<br>56.3; 6.9; 25.4<br>m.p. 80-81°C |
| 83 | H | $CH_3$ | $CH_3$ | $O_2S$ | 46.9; 5.4; 24.9<br>46.9; 5.3; 25.1<br>m.p. 219-224°C |
| 84 | $CH(CH_3)_2$ | $CH_2CH_3$ | H | S | (as $H_2SO_4$ Salt):<br>43.7; 6.0; 18.0<br>43.1; 6.0; 18.4<br>m.p. 193-194°C |
| 85 | $CH(CH_3)_2$ | $CH_2CH_3$ | H | O | (as $H_2SO_4$ Salt):<br>45.0; 6.2; 18.8<br>44.9; 6.3; 19.1<br>m.p. 189-190°C |
| 86 | $(CH_2)_2CH_3$ | $CH_2CH_3$ | H | O | (as $H_2SO_4$ Salt):<br>45.0; 6.2; 18.8<br>44.9; 6.3; 19.1<br>m.p. 170-172°C |

Table III (continued)

| Ex. No. | Compound of Formula IA; Product of Formula IB: $R_2$ | $R_5$ | $R_8$ | X | Product of Formula IB Calculated: %C; %H; %N Found: %C; %H; %N |
|---|---|---|---|---|---|
| 87 | $(CH_2)_2CH_3$ | $CH_2CH_3$ | H | S | (as $H_2SO_4$ Salt):<br>43.2; 6.0; 18.0<br>43.0; 6.1; 18.3<br>m.p. 152-153°C |
| 88 | $(CH_2)_2CH_3$ | $CH_3(CH_2)_2$ | H | S | (as $H_2SO_4$ Salt):<br>44.6; 6.2; 17.4<br>44.7; 6.4; 17.5<br>m.p. 143-145°C |
| 89 | $(CH_2)_2CH_3$ | $(CH_2)_2CH_3$ | H | O | (as $H_2SO_4$ Salt):<br>46.5; 6.5; 18.1<br>46.6; 6.7; 18.4<br>m.p. 169-171°C |
| 90 | $CH(CH_3)_2$ | $(CH_2)_2CH_3$ | H | S | (as $H_2SO_4$ Salt):<br>44.6; 6.2; 17.4<br>44.8; 6.4; 17.6<br>m.p. 172-173°C |
| 91 | $CH(CH_3)_2$ | $CH_3(CH_2)_2$ | H | O | (as $H_2SO_4$ Salt):<br>46.5; 6.5; 18.1<br>46.4; 6.7; 18.3<br>m.p. 156-157°C |
| 92 | $CH_2CH_3$ | $CH(CH_3)_2$ | H | O | 61.1; 7.7; 25.4<br>61.0; 7.9; 25.9<br>m.p. 128-129°C |

Table III (continued)

| Ex. No. | Compound of Formula IA; Product of Formula IB $R_2$ | $R_5$ | $R_8$ | X | Product of Formula IB Calculated: %C; %H; %N Found: %C; %H; %N |
|---|---|---|---|---|---|
| 93 | $(CH_2)_2CH_3$ | $CH(CH_3)_2$ | H | O | 62.3; 8.0; 24.2<br>61.3; 8.1; 24.0<br>m.p. 85-86°C |
| 94 | $CH_3$ | $CH(CH_3)_2$ | H | S | 56.3; 6.9; 25.3<br>56.1; 6.9; 25.8<br>m.p. 170-172°C |
| 95 | $CH_2CH_3$ | $(CH_3)_2CH$ | H | S | 57.7; 7.3; 24.0<br>57.3; 7.1; 24.3<br>m.p. 142-143°C |
| 96 | H | $CH(CH_3)_2$ | H | O | (as $H_2SO_4$ Salt):<br>41.7; 5.5; 20.3<br>41.0; 5.2; 20.3<br>m.p. 179-182°C |

Example 97

Preparation of 2-n-Propyl-7-(4-thiomorpholino)-1,2,4-triazolo[1,5-c]pyrimidine

A mixture of 8.00 g (37.9 mmole) of 4-hydrazino-6-(4-thiomorpholino)pyrimidine and 20 ml of trimethyl ortho-*n*-butyrate was heated at its reflux temperature for about 60 hours, cooled, and the solid was isolated by filtration. The product was washed with diethyl ether, then recrystallized twice from a benzene-hexane mixture accompanied by treatment with decolorizing charcoal to provide off-white 2-n-propyl-7-(4-thiomorpholino)-1,2,4-triazolo[1,5-c]pyrimidine, m.p. 137—138°C. Most (4.6 g, 17.5 mmole) of this product was dissolved in 100 ml of ethanol and 1.75 g (17.1 mmole) of sulfuric acid was added. The solution was diluted to 300 ml with diethyl ether and allowed to stand for two hours. The precipitate was collected by filtration, washed with diethyl ether and dried to provide the dihydrogen sulfate salt, m.p. 134—137°C. Analysis: Calculated for $C_{12}H_{17}N_5S \cdot H_2SO_4$: %C, 39.9; %H, 5.3; %N, 19.4; Found: %C, 39.5; %H, 5.2; %N, 19.5. The structural assignment was confirmed by infrared spectral analysis

Examples 98—103

Using the method of Example 97, the indicated intermediates of Formula VIII were reacted with the indicated orthoesters to provide the indicated compounds of Formula IB (Table IV).

Table IV

| Example Number | Intermediate of Formula VIII $R_5$ | $R_8$ | X | Orthoester | Product of Formula IB $R_2$ | $R_5$ | $R_8$ | X | Calculated: %C; %H; %N / Found: %C; %H; %N (m.p. in C) |
|---|---|---|---|---|---|---|---|---|---|
| 98 | H | H | O | trimethyl orthoisobutyrate | $CH(CH_3)_2$ | H | H | O | 58.3; 6.9; 28.3<br>58.1; 6.9; 28.5<br>(154–155) |
| 99 | H | H | S | triethyl orthoacetate | $CH_3$ | H | H | S | 51.0; 5.6; 29.8<br>50.9; 5.5; 30.1<br>(145–148) |
| 100 | H | H | S | triethyl orthopropionate | $CH_2CH_3$ | H | H | S | 53.0; 6.1; 28.1<br>52.7; 6.0; 28.3<br>(125–126) |
| 101 | H | H | S | trimethyl orthobutyrate | $CH_2CH_2CH_3$ | H | H | S | 54.7; 6.5; 26.6<br>54.6; 6.4; 26.7<br>(137–138) |

Table IV (continued)

| Example Number | Intermediate of Formula VIII $R_5$ | $R_8$ | X | Orthoester | Product of Formula IB $R_2$ | $R_5$ | $R_8$ | X | Calculated: %C; %H; %N<br>Found: %C; %H; %N<br>(m.p. in C) |
|---|---|---|---|---|---|---|---|---|---|
| 102 | H | H | O | triethyl orthoformate | H | H | H | O | 52.7; 5.4; 34.1<br>52.2; 5.3; 34.2<br>(202-204) |
| 103 | H | H | O | triethyl orthopropionate | $CH_2CH_3$ | H | H | O | 56.6; 6.5; 30.0<br>56.5; 6.3; 30.2<br>(164-165) |

Example 104

Preparation of 2,5-Di(isopropyl)-7-(4-thiomorpholino)-1,2,4-triazolo[1,5-c]pyrimidine

A mixture of 4.00 g (15.8 mmole) of 4-hydrazino-2-isopropyl-6-(4-thiomorpholino)pyrimidine, 2.40 g (16.2 mmole) of trimethyl isobutyrate, 0.95 g (15.8 mmole) of acetic acid and 40 ml of diethyl sulfoxide was heated at 120°C for about 65 hours and then poured into 200 ml of ice water. The mixture was basified with 10 percent aqueous sodium hydroxide and extracted four times with 50 ml of chloroform. The extracts were washed six times with 150 ml of water, dried over magnesium sulfate and evaporated to provide a dark oil. Nuclear magnetic resonance spectral analysis indicated that the oil was chiefly the desired product. The product was chromatographed on 70 g of silica gel, eluting sequentially with 600 ml of dichloromethane, one liter of 50:50 ethyl acetate:dichloromethane, and ethyl acetate, 100 ml fractions being taken. Fractions 7, 8 and 9 provided 3.35 g (70%) of brown oil. The oil was dissolved in 50 ml of ethanol, and sulfuric acid (1.09 g, 10.7 mmole) and 200 ml of diethyl ether were then added sequentially. The precipitate was collected by filtration, washed with diethyl ether and dried. The product was off-white solid 2,5-di(isopropyl)-7-(4-thiomorpholino)-1,2,4-triazolo[1,5-c]pyrimidine dihydrogen sulfate, m.p. 192—194°C. Analysis: Calculated for $C_{15}H_{23}N_5S \cdot H_2SO_4$: %C, 44.6; %H, 6.3; %N, 17.4; Found: %C, 44.6; %H, 6.3; %N, 17.5. The structure was confirmed by infrared and nuclear magnetic resonance spectral analyses.

Examples 105—127

Using the method of Example 104, the indicated intermediates of Formula VIII were reacted with the indicated orthoesters to provide the indicated compounds of Formula IB (Table V).

Table V

| Example Number | Intermediate of Formula VIII: $R_5$ | $R_8$ | X | Orthoester | Product of Formula IB: $R_2$ | $R_5$ | $R_8$ | X | Calculated: %C; %H; %N<br>Found: %C; %H; %N<br>(m.p. in C) |
|---|---|---|---|---|---|---|---|---|---|
| 105 | $CH_2CH_3$ | H | S | trimethyl orthoisovalerate | $CH_2CH(CH_3)_2$ | $CH_2CH_3$ | H | S | (as $H_2SO_4$ Salt):<br>44.6; 6.2; 17.4<br>44.5; 6.5; 17.6<br>(155–156) |
| 106 | $CH(CH_3)_2$ | H | S | trimethyl orthoisovalerate | $CH_2CH(CH_3)_2$ | $CH(CH_3)_2$ | H | S | (as $H_2SO_4$ Salt):<br>46.0; 6.5; 16.8<br>45.9; 6.7; 16.9<br>(169–170) |
| 107 | $CH_2CH_3$ | H | S | trimethyl orthovalerate | $(CH_2)_3CH_3$ | $CH_2CH_3$ | H | S | (as $H_2SO_4$ Salt):<br>44.6; 6.2; 17.4<br>44.3; 6.2; 17.7<br>(132–135) |

Table V (continued)

| Example Number | Intermediate of Formula VIII: $R_5$ | $R_8$ | X | Orthoester | Product of Formula IB: $R_2$ | $R_5$ | $R_8$ | X | Calculated: %C; %H; %N<br>Found: %C; %H; %N<br>(m.p. in C) |
|---|---|---|---|---|---|---|---|---|---|
| 108 | $(CH_2)_2CH_3$ | H | S | trimethyl orthovalerate | $(CH_2)_3CH_3$ | $(CH_2)_2CH_3$ | H | S | (as $H_2SO_4$ Salt):<br>46.0; 6.5; 16.8<br>46.0; 6.4; 17.1<br>(152–153) |
| 109 | $CH_2CH(CH_3)_2$ | H | S | trimethyl orthoisobutyrate | $CH(CH_3)_2$ | $CH_2CH(CH_3)_2$ | H | S | 60.1; 7.9; 21.9<br>59.7; 7.9; 21.7<br>(94–97) |
| 110 | $CH_2CH(CH_3)_2$ | H | O | triethyl orthopropionate | $CH_2CH_3$ | $CH_2CH(CH_3)_2$ | H | O | 62.2; 8.0; 24.2<br>62.0; 7.7; 24.2<br>(119–122) |
| 111 | $CH_2CH(CH_3)_2$ | H | O | trimethyl orthoisobutyrate | $CH(CH_3)_2$ | $CH_2CH(CH_3)_2$ | H | O | 63.3; 8.3; 23.1<br>63.2; 8.0; 23.1<br>(93–95) |

Table V (continued)

| Example Number | Intermediate of Formula VIII: $R_5$ | $R_8$ | X | Orthoester | Product of Formula IB: $R_2$ | $R_5$ | $R_8$ | X | Calculated: %C; %H; %N / Found: %C; %H; %N (m.p. in C) |
|---|---|---|---|---|---|---|---|---|---|
| 112 | $CH_2CH(CH_3)_2$ | H | O | trimethyl orthovalerate | $(CH_2)_3CH_3$ | $CH_2CH(CH_3)_2$ | H | O | 64.3; 8.6; 22.1<br>63.7; 8.2; 21.9<br>(85-88) |
| 113 | $(CH_2)_3CH_3$ | H | S | triethyl orthoacetate | $CH_3$ | $(CH_2)_3CH_3$ | H | S | 57.7; 7.7; 24.0<br>57.3; 7.3; 24.1<br>(123-126) |
| 114 | $(CH_2)_3CH_3$ | H | S | trimethyl orthobutyrate | $(CH_2)_2CH_3$ | $(CH_2)_3CH_3$ | H | S | 60.3; 7.9; 22.0<br>59.9; 8.0; 21.9<br>(66-68) |
| 115 | $(CH_2)_3CH_3$ | H | S | trimethyl orthoisovalerate | $CH_2CH(CH_3)_2$ | $(CH_2)_3CH_3$ | H | S | 61.4; 8.2; 21.1<br>60.9; 8.3; 21.1<br>(50-53) |

Table V (continued)

| Example Number | Intermediate of Formula VIII: $R_5$ | $R_8$ | X | Orthoester | Product of Formula IB: $R_2$ | $R_5$ | $R_8$ | X | Calculated: %C; %H; %N<br>Found: %C; %H; %N<br>(m.p. in C) |
|---|---|---|---|---|---|---|---|---|---|
| 116 | $CH_2CH(CH_3)_2$ | H | O | triethyl orthoformate | H | $CH_2CH(CH_3)_2$ | H | O | 59.7; 7.3; 26.8<br>59.6; 7.2; 26.9<br>(123-126) |
| 117 | $(CH_2)_3CH_3$ | H | S | triethyl orthopropionate | $CH_2CH_3$ | $(CH_2)_3CH_3$ | H | S | 59.0; 7.6; 22.9<br>59.2; 7.7; 23.3<br>(110-112) |
| 118 | $CH_2CH_3$ | H | O | trimethyl orthovalerate | $(CH_2)_3CH_3$ | $CH_2CH_3$ | H | O | 62.3; 8.0; 24.2<br>62.1; 8.0; 24.3<br>(87-89) |
| 119 | $(CH_2)_2CH_3$ | H | O | trimethyl orthovalerate | $(CH_2)_3CH_3$ | $(CH_2)_2CH_3$ | H | O | 63.3; 8.3; 23.1<br>63.1; 8.2; 23.3<br>(105-106) |

Table V (continued)

| Example Number | Intermediate of Formula VIII: $R_5$ | $R_8$ | X | Orthoester | Product of Formula IB: $R_2$ | $R_5$ | $R_8$ | X | Calculated: %C; %H; %N<br>Found: %C; %H; %N<br>(m.p. in C) |
|---|---|---|---|---|---|---|---|---|---|
| 120 | $(CH_2)_3CH_3$ | H | O | triethyl orthoacetate | $CH_3$ | $(CH_2)_3CH_3$ | H | O | 61.1; 7.7; 25.4<br>61.0; 7.8; 25.3<br>(132-133) |
| 121 | $(CH_2)_3CH_3$ | H | O | triethyl orthopropionate | $CH_2CH_3$ | $(CH_2)_3CH_3$ | H | O | 62.3; 8.0; 24.2<br>62.1; 8.1; 24.2<br>(105-106) |
| 122 | $(CH_2)_3CH_3$ | H | O | trimethyl orthobutyrate | $(CH_2)_2CH_3$ | $(CH_2)_3CH_3$ | H | O | 63.3; 8.3; 23.1<br>63.3; 8.4; 23.2<br>(98-99) |
| 123 | $(CH_2)_3CH_3$ | H | O | trimethyl orthoisovalerate | $CH_2CH(CH_3)_2$ | $(CH_2)_3CH_3$ | H | O | 64.3; 8.6; 22.1<br>64.2; 8.4; 22.0<br>(73-75) |

Table V (continued)

| Example Number | Intermediate of Formula VIII: $R_5$ | $R_8$ | X | Orthoester | Product of Formula IB: $R_2$ | $R_5$ | $R_8$ | X | Calculated: %C; %H; %N<br>Found: %C; %H; %N<br>(m.p. in C) |
|---|---|---|---|---|---|---|---|---|---|
| 124 | $CH_2CH(CH_3)_2$ | H | S | triethyl orthopropionate | $CH_2CH_3$ | $CH_2CH(CH_3)_2$ | H | S | 59.0; 7.6; 22.9<br>59.0; 7.2; 23.1<br>(124-126) |
| 125 | $(CH_2)_2CH_3$ | H | O | triethyl orthoisovalerate | $CH_2CH(CH_3)_2$ | $(CH_2)_2CH_3$ | H | O | 63.3; 8.3; 23.1<br>63.1; 7.9; 23.2<br>(53-55) |
| 126 | $CH_2CH(CH_3)_2$ | H | S | triethyl orthoformate | H | $CH_2CH(CH_3)_2$ | H | S | 56.3; 6.9; 25.2<br>56.3; 6.9; 25.4<br>(134-136) |
| 127 | $CH_2CH_3$ | H | O | trimethyl orthoisovalerate | $CH_2CH(CH_3)_2$ | $CH_2CH_3$ | H | O | 62.3; 8.0; 24.2<br>62.3; 7.9; 24.3<br>(50-52) |

Example 128

Part A Preparation of 4-Chloro-2-methyl-6-(4-morpholino)pyrimidine by Scheme I, Step (5)

A solution of 5.00 g (31.7 mmole) of 4,6-dichloro-2-methylpyrimidine and 6.00 g (68.9 mmole) of morpholine in 50 ml of water was heated on a steam cone for about 18 hours. The mixture was diluted with water and cooled. The resulting white solid was separated by filtration, washed with water and dried to provide 4.84 g (72%) of 4-chloro-2-methyl-6-(4-morpholino)pyrimidine. The structural assignment was confirmed by infrared and nuclear magnetic resonance spectral analyses.

Part B Preparation of 4-Hydrazino-2-methyl-6-(4-morpholino)pyrimidine by Scheme I, Step (6)

To a mixture of 4.70 g (22 mmole) of 4-chloro-2-methyl-6-(4-morpholino)pyrimidine in 50 ml of ethanol was added 2.2 g (44 mmole) of hydrazine hydrate and the mixture was heated at its reflux temperature for 16 hours. Cooling provided a precipitate which was separated by filtration and washed with ethanol to provide 3.15 g (68%) of white solid 4-hydrazino-2-methyl-6-(4-morpholino)pyrimidine. The structural assignment of the product was confirmed by infrared and nuclear magnetic resonance spectral analyses and comparison with the same compound made in Example 9.

Examples 129—131

Using the method of Example 128, Part A, the indicated intermediate of Formula V was reacted with the indicated amine of Formula VIA to provide the indicated intermediate of Formula VII. The intermediate of Formula VII was then reacted with hydrazine hydrate in accordance with the method of Example 128, Part B, to provide the indicated intermediate of Formula VIII (Table VI).

Table VI

| Example Number | Intermediate of Formula V | Heterocyclic Amine | Intermediate of Formula VII | Intermediate of Formula VIII |
|---|---|---|---|---|
| 129 | 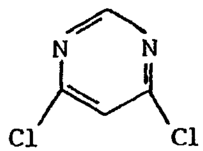 | 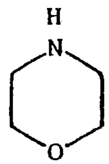 | 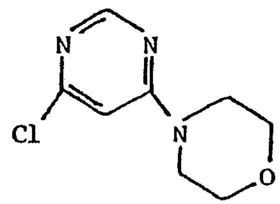 m.p. 147.5-150 C, White Solid | 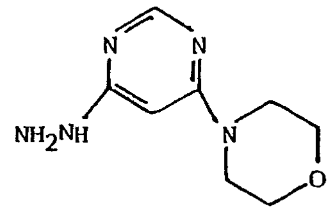 White Solid |
| 130 | 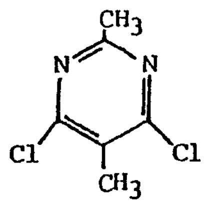 | 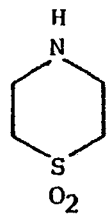 | 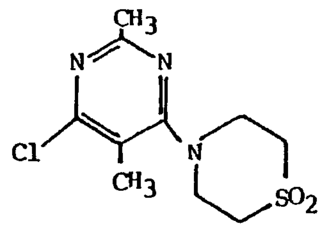 m.p. 210-220°C, White Solid | 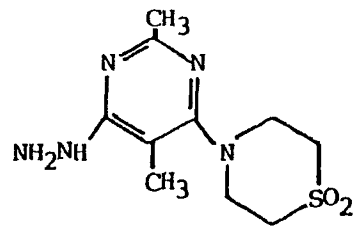 m.p. 232-234°C, White Solid |

Table VI (continued)

| Example Number | Intermediate of Formula V | Heterocyclic Amine | Intermediate of Formula VII | Intermediate of Formula VIII |
|---|---|---|---|---|
| 131 | 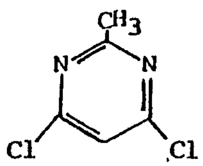 | 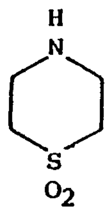 | 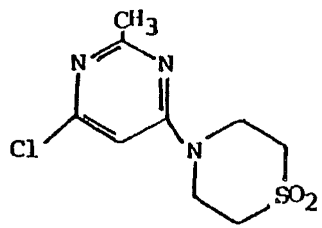 | 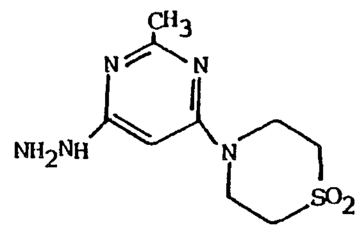 |
| | | | m.p. about 200°C | m.p. 250–253°C, White Solid |

Example 132
Preparation According to Scheme II of 5-Methylthio-7-(4-morpholino)1,2,4-triazolo[4,3-c]pyrimidine and 5,7-Bis(4-morpholno)-1,2,4-triazolo[1,5-c]pyrimidine

Part A Preparation of 4-Hydrazinyl-2-methylthio-6-(4-morpholino)pyrimidine according to Scheme II, Step (1)

To a solution of 3.0 g (15.7 mmole) of 4-chloro-6-hydrazinyl-2-methylthiopyrimidine in 50 ml of water was added 2.8 g (32.3 mmole) of morpholine, and the mixture was heated at reflux for two days. Cooling gave a precipitate which was separated by filtration, washed with water and dried to provide off-white solid 4-hydrazinyl-2-methylthio-6-(4-morpholino)pyrimidine, m.p. 134—144°C. The structural assignment was confirmed by infrared and nuclear magnetic resonance spectral analyses.

Part B Preparation of 5-Methylthio-7-(4-morpholino)-1,2,4-triazolo[4,3-c]pyrimidine according to Scheme II, Step (2)

A mixture of 24.75 g (103 mmole) of 4-hydrazinyl-2-methylthio-6-(4-morpholino)pyrimidine and 200 ml of triethyl orthoformate was heated at 120°C in an open flask for 60 hours. The mixture was cooled, then diluted with 300 ml of diethyl ether. The precipitate was separated by filtration, washed with ether and dried to provide 5-methylthio-7-(4-morpholino)-1,2,4-triazolo[4,3-c]pyrimidine, m.p. 212—213°C after two recrystallizations from chloroform-hexane.

Part C Preparation of 5,7-Bis(4-morpholino)-1,2,4-triazolo[1,5-c]pyrimidine according to Scheme II, Step (3)

A mixture of 7.10 g (28.3 mmole) of 5-methylthio-7-(4-morpholino)1,2,4-triazolo[1,5-c]pyrimidine and 35 ml of morpholine was heated at reflux for 16 hours. Cooling of the mixture provided a solid. The mixture was diluted to a total volume of 100 ml with diethyl ether, cooled and the product separated by filtration. Recrystallization with treatment with decolorizing charcoal from a mixture of benzene and hexane (1:1) provided white solid 5,7-bis(4-morpholino)-1,2,4-triazolo[1,5-c]pyrimidine, m.p. 176—177°C. Analysis: Calculated for $C_{13}H_{18}N_6O_2$: %C, 53.8; %H, 6.2; %N, 29.0; Found: %C, 53.8; %H, 6.1; %N, 28.9. The structural assignment was confirmed by infrared and nuclear magnetic resonance spectral analyses.

Examples 133—135

Using the method of Example 132, Part A, the indicated intermediates of Formula XI were prepared from 4-chloro-6-hydrazino-2-methylthiopyrimidine and the indicated amine of Formula VIA (Table VII). The structures of the intermediates of Formula XI were confirmed by infrared and nuclear magnetic resonance spectral analyses.

Table VII

| Example | Amine Reactant of Formula VIA | Intermediate of Formula XI |
|---|---|---|
| 133 | 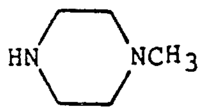 | 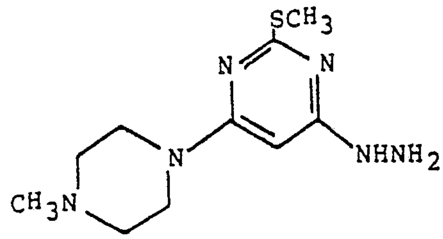 |
| 134 | 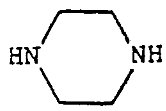 | 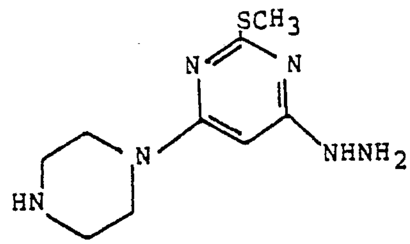 |
| 135 | 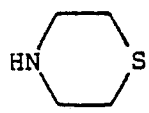 | 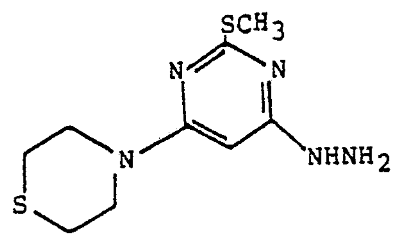 |

Examples 136—138

Using the method of Example 132, Part B, the indicated compounds of Formula IA were prepared from the indicated intermediate of Formula XI (Table VIII). The structures of the compounds of Formula IA were confirmed by infrared and nuclear magnetic resonance spectral analyses.

Table VIII

| Example | Intermediate of Formula XI | Compound of Formula IA |
|---|---|---|
| 136 | Example 133 | 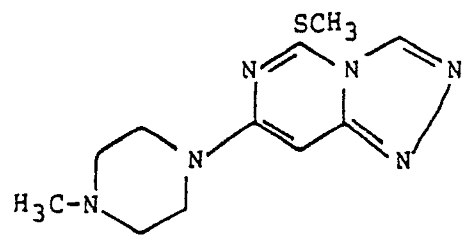 |
| 137 | Example 134 | 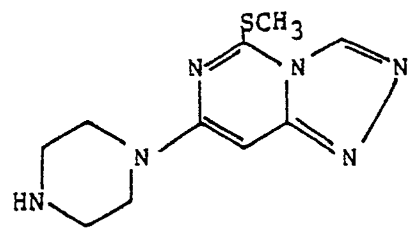 |
| 138 | Example 135 | 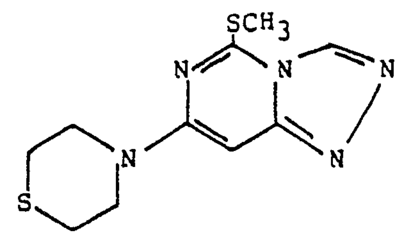 |

Examples 139—141

Using the method of Example 132, Part C, the indicated compounds of Formula IB were prepared from the indicated compound of Formula IA and the indicated amine of Formula VIA (Table IX). Chromatographic separations were used to obtain the desired [1,5-c] isomers.

Table IX

| Example | Compound of Formula IA | Amine of Formula VIA | Product of Formula IB | Calculated: %C; %H; %N<br>Found: %C; %H; %N |
|---|---|---|---|---|
| 139 | Example 136 | 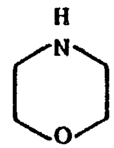 | 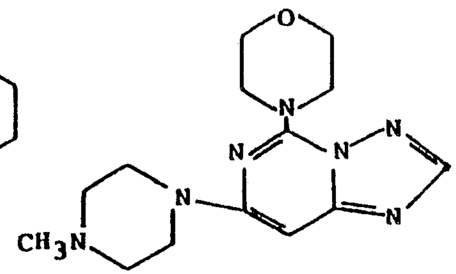 | 55.4; 7.0; 32.3<br>55.9; 6.9; 31.8<br>(m.p. 142-143.5°C) |
| 140 | Example 137 | 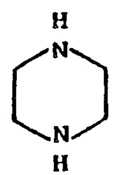 | 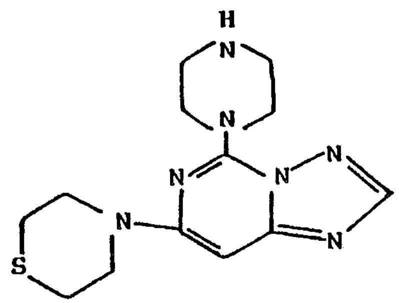 | 51.1; 6.3; 32.1<br>51.5; 6.3; 31.8<br>(m.p. 127-130°C) |
| 141 | Example 138 | 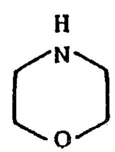 | 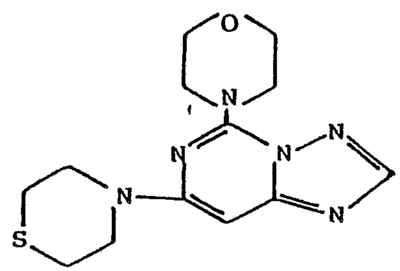 | 51.0; 5.9; 27.4<br>50.7; 5.9; 27.6<br>(m.p. 169-170°C) |

Example 142

The Preparation of 2,7-Dimethyl-5-(1-piperazino)-1,2,4-triazolo[1,5-c]pyrimidine according to Scheme III

A solution of the known compound 5-chloro-2,7-dimethyl-1,2,4-triazolo[1,5-c]pyrimidine (2.0 g, 11 mmole) in 50 ml of dioxane was added dropwise to a suspension of 120 g (140 mmole) of piperazine in 75 ml of dioxane. After stirring for 3 hours at 20°C, the mixture was diluted with 150 ml of water and then extracted with three 150 ml portions of chloroform. The extracts were washed with two 150 ml portions of water and two 100 ml portions of saturated sodium chloride solution and were then dried over magnesium sulfate. The extracts were evaporated to provide a residue which was recrystallized with treatment with decolorizing charcoal from a benzene-hexane mixture (1:3) to provide white solid 2,7-dimethyl-5-(1-piperazino)-1,2,4-triazolo[1,5-c]pyrimidine, m.p. 108—110°C. Analysis: Calculated for $C_{11}H_{16}N_6$: %C, 56.9; %H, 6.9; %N, 36.2; Found: %C, 57.0; %H, 6.9; %N, 35.6.

Examples 143—156

Using the method of Example 142, the indicated compounds of Formula IB were prepared from the indicated intermediates of Formula XIV and the indicated amines of Formula VIA (Table X).

Table X

| Example | Intermediate of Formula XIV $R_2$ | $R_7$ | $R_8$ | Amine of Formula VIA | Product of Formula IB $R_2$ | $R_5$ | $R_7$ | $R_8$ | Calculated: %C; %H; %N<br>Found: %C; %H; %N<br>(m.p. in C) |
|---|---|---|---|---|---|---|---|---|---|
| 143 | $CH_3$ | $CH_3$ | H | 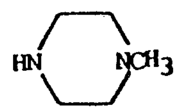 | $CH_3$ | 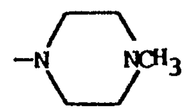 | $CH_3$ | H | 58.5; 7.4; 34.1<br>58.8; 7.3; 34.8<br>(106.5-107.5) |
| 144 | $CH_3$ | $CH_3$ | H | 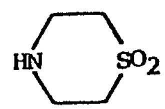 | $CH_3$ | 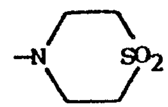 | $CH_3$ | H | 47.0; 5.4; 24.9<br>47.0; 5.3; 25.4<br>(201-203) |
| 145 | $CH_3$ | $CH_3$ | H | 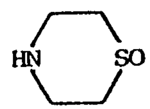 | $CH_3$ | 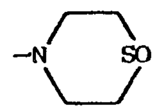 | $CH_3$ | H | 49.8; 5.7; 26.4<br>50.0; 5.6; 26.8<br>(186-188) |
| 146 | $CH_3$ | $CH_3$ | H | 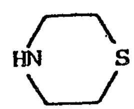 | $CH_3$ | 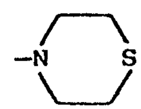 | $CH_3$ | H | 53.0; 6.1; 28.1<br>53.0; 6.1; 28.7<br>(133-134) |

Table X (continued)

| Example | Intermediate of Formula XIV: $R_2$ | Intermediate of Formula XIV: $R_7$ | Amine of Formula VIA | Product of Formula IB: $R_2$ | Product of Formula IB: $R_5$ | Product of Formula IB: $R_7$ | Product of Formula IB: $R_8$ | Calculated: %C; %H; %N<br>Found: %C; %H; %N<br>(m.p. in C) |
|---|---|---|---|---|---|---|---|---|
| 147 | $CH_3$ | $CH_3$ | 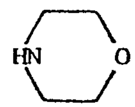 | $CH_3$ | 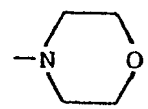 | $CH_3$ | H | 56.6; 6.5; 30.0<br>56.6; 6.4; 30.5<br>(142–144) |
| 148 | H | $CH_3$ | 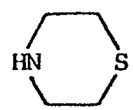 | H | 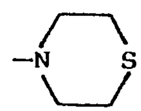 | $CH_3$ | H | 51.0; 5.6; 29.8<br>50.7; 5.6; 29.8<br>(99.5–100) |
| 149 | H | $CH_3$ | 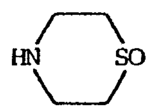 | H | 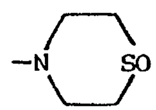 | $CH_3$ | H | 48.7; 5.2; 27.9<br>48.1; 5.2; 28.3<br>(188–190) |
| 150 | H | $CH_3$ | 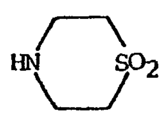 | H | 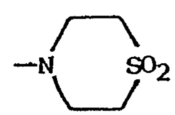 | $CH_3$ | H | 44.9; 4.9; 26.2<br>44.7; 5.0; 26.3<br>(222–223) |

Table X (continued)

| Example | Intermediate of Formula XIV: $R_2$ | Intermediate of Formula XIV: $R_7$ | Amine of Formula VIA | Product of Formula IB: $R_2$ | Product of Formula IB: $R_5$ | Product of Formula IB: $R_7$ | Product of Formula IB: $R_8$ | Calculated: %C; %H; %N<br>Found: %C; %H; %N<br>(m.p. in C) |
|---|---|---|---|---|---|---|---|---|
| 151 | $CH_2CH_3$ | $CH_3$ | 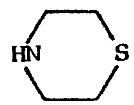 | $CH_2CH_3$- | 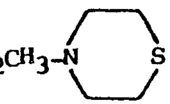 | $CH_3$ | H | 54.7; 6.5; 26.7<br>54.7; 6.7; 27.2<br>(106-107) |
| 152 | $CH_2CH_3$ | $CH_3$ | 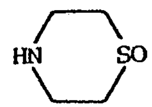 | $CH_2CH_3$- | 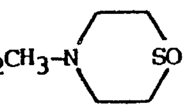 | $CH_3$ | H | 51.6; 6.1; 25.1<br>51.5; 6.3; 25.0<br>(146-148) |
| 153 | $CH_2CH_3$ | $CH_3$ | 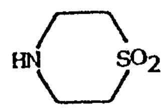 | $CH_2CH_3$- | 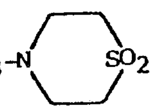 | $CH_3$ | H | 48.8; 5.8; 23.7<br>48.8; 6.0; 24.3<br>(183-186) |
| 154 | $CH_3$ | H | 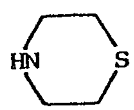 | $CH_3$ | 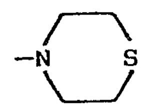 | H | H | 51.0; 5.6; 29.8<br>51.0; 5.5; 30.0<br>(83-85) |

Table X

| Example | Intermediate of Formula XIV: $R_2$ | Intermediate of Formula XIV: $R_7$ | Amine of Formula VIA | Product of Formula IB: $R_2$ | Product of Formula IB: $R_5$ | Product of Formula IB: $R_7$ | Product of Formula IB: $R_8$ | Calculated: %C; %H; %N<br>Found: %C; %H; %N<br>(m.p. in C) |
|---|---|---|---|---|---|---|---|---|
| 155 | $CH_3$ | H | 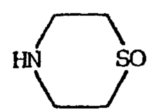 | $CH_3$ | 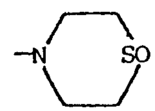 | H | H | 47.8; 5.2; 27.9<br>48.0; 5.3; 28.0<br>(150–153) |
| 156 | $CH_3$ | H | 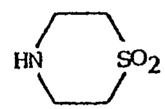 | $CH_3$ | 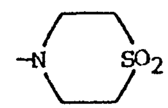 | H | H | 44.9; 4.9; 26.2<br>45.0; 4.9; 25.9<br>(183–185) |

Example 157

Preparation of 7-Methyl-5-(1-piperazinyl)-1,2,4-triazolo[4,3-c]pyrimidine and 7-Methyl-5-(1-piperazinyl)-1,2,4-triazolo[1,5-c]pyrimidine by Scheme IV

A mixture of 6.00 g (33.3 mmole) of 7-methyl-5-methylthio-1,2,4-triazolo[4,3-c]pyrimidine, 30.0 g (0.35 mmole) of piperazine and 250 ml of dioxane was refluxed under nitrogen for six days. The mixture was cooled and concentrated *in vacuo.* The residue obtained was dissolved in 150 ml of water, and the solution was extracted four times with 150 ml portions of chloroform. The extracts were washed thrice with 150 ml portions of water and twice with 150 ml portions of sodium chloride solution and were dried over magnesium sulfate. Evaporation provided a yellow solid which was taken up in 150 ml of chloroform, filtered and chromatographed on a high pressure liquid chromatograph, eluting with methanol-ethyl acetate (1:1). Infrared and nuclear magnetic resonance spectral analyses showed fractions 2 and 3 to be 7-methyl-5-(1-piperazino)-1,2,4-triazolo[1,5]pyrimidine, m.p. 92—95°C. Analysis: Calculated for $C_{10}H_{14}N_6$: %C, 55.0; %H, 6.5; %N, 38.5; Found: %C, 54.8; %H, 6.4; %N, 38.3. Fraction 4 contained about 15% of 7-methyl-5-(1-piperazino)-1,2,4-triazolo[4,3-c]pyrimidine, m.p. 136—139°C. Analysis Calculated for $C_{10}H_{14}N_6$: %C, 55.0; %H, 6.5; %N, 38.5. Found: %C, 54.5; %H, 6.4; %N, 37.9.

Example 158

Preparation of 7-Methyl-5-(4-piperazinyl)-1,2,4-triazolo[4,3-c]pyrimidine and 7-Methyl-5-(4-methyl-1-piperazinyl)-1,2,4-triazolo[1,5-c]pyrimidine

Using the method of Example 157, 7-methyl-5-methylthio-1,2,4-triazolo[4,3-c]pyrimidine was reacted with N-methylpiperazine to provide a mixture of 7-methyl-5-(4-methyl-1-piperazinyl)1,2,4-triazolo[4,3-c]-pyrimidine, m.p. 170—171°C (Analysis: Calculated for $C_{11}H_{14}N_6$: %C, 56.9; %H, 6.9; %N, 36.2; Found: %C, 57.0; %H, 6.9; %N, 35.9) and 7-methyl-5-(4-methyl-1-piperazinyl)-1,2,4-triazolo[1,5-c]pyrimidine, m.p. 95—98°C. These compounds were separated by high pressure liquid chromatography using 5% methanol in ethyl acetate which contained a small amount of diethylamine.

Example 159

Preparation of 7-Methyl-5-(4-morpholino)-1,2,4-triazolo[4,3-c]pyrimidine and 7-Methyl-5-(4-morpholino)-1,2,4-triazolo[1,5-c]pyrimidine

A mixture of 6.0 g (33 mmole) of 7-methyl-5-methylthio-1,2,4-triazolo[4,3-c]pyrimidine and 15 ml of morpholine was heated at reflux for 19 hours, cooled and diluted with diethyl ether and hexane. The solid product was separated by filtration and chromatographed on florisil, eluting sequentially with benzene, 10% ethyl acetate in benzene, 50% ethyl acetate in benzene, and ethyl acetate. Early fractions were recrystallized from a benzene-hexane mixture to provide 7-methyl-5-(4-morpholino)-1,2,4-triazolo[1,5-c]-pyrimidine, m.p. 113—114°C. Later fractions were recrystallized from an ethyl acetate-hexane mixture with treatment with decolorizing charcoal to provide 7-methyl-5-(4-morpholino)-1,2,4-triazolo[4,3-c]pyrimidine, m.p. 209—210°C. Analysis: Calcuated for $C_{10}H_{13}N_5O$: %C, 54.8; %H, 6.0; %N, 31.9; Found: %C, 55.1; %H, 5.9; %N, 31.8.

Example 160

To a solution of 3.00 g (10.8 mmole) of 2,5-diethyl-7-(4-thiomorpholino)-1,2,4-triazolo[1,5-c]pyrimidine in 60 ml of warm ethanol was added 1.05 g (10.7 mmole) of concentrated sulfuric acid. The solution was diluted to 250 ml with diethyl ether and let stand for two hours. The precipitated product was separated by filtration and washed with ether and dried to provide 2,5-diethyl-7-(4-thiomorpholino)-1,2,4-triazolo[1,5-c]-pyrimidine dihydrogen sulfate as a white solid, m.p. 178—180°C. Analysis: Calculated for $C_{13}H_{19}H_5S\cdot H_2SO_4$: %C, 41.5; %H, 5.6; %N, 18.7; Found: %C, 41.7; %H, 5.7; %N, 19.0.

Example 161

To a solution of 1.75 g (6.31 mmole) of 2,5-diethyl-7-(4-thiomorpholino)-1,2,4-triazolo[1,5-c]pyrimidine in 20 ml of dioxane was added 1.0 ml of 6.4 M hydrogen chloride (6.4 mmole) in ethanol. The solution was diluted with 100 ml of diethyl ether and allowed to stand several hours. The white solid was separated by filtration and dried to provide 2,5-diethyl-7-(4-thiomorpholino)-1,2,4-triazolo[1,5-c]pyrimidine hydrochloride hydrate, m.p. 113—114°C. Analysis: Calculated for $C_{13}H_{19}N_5S\cdot HCl\cdot H_2O$: %C, 47.0; %H, 6.7; %N, 21.1; Found: %C, 46.9; %H, 6.8; %N, 21.3.

Example 162

To a solution of 2.00 g (7.21 mmole) of 2,5-diethyl-7-(4-thiomorpholino)-1,2,4-triazolo[1,5-c]pyrimidine in 20 ml of warm ethanol was added 0.85 g (7.4 mmole) of phosphoric acid. The solution was diluted with 75 ml of diethyl ether and allowed to stand. After a few minutes the white solid was separated by filtration, washed with diethyl ether and dried to provide 2,5-diethyl-7-(4-thiomorpholino)-1,2,4-triazolo[1,5-c]-pyrimidine trihydrogen phosphate hydrate, m.p. 154—155°C. Analysis: Calculated for $C_{13}H_{19}N_5S\cdot H_3PO_4H_2O$: %C, 36.4; %H, 6.6; %N, 16.3; Found: %C, 36.5; %H, 5.8; %N, 16.6

Examples 163—164

Using the method of Example 1, Part E, the indicated amines of Formula VIA were reacted with the indicated known 4-chloro-2-alkyl-6-hydrazinylpyrimidines of Formula VI to provide the novel intermediates of Formula VIII (Table XI).

Table XI

| Example | Amine Intermediate | Pyrimidine Intermediate of Formula VI | Intermediate of Formula VIII | Melting Point (in°C) |
| --- | --- | --- | --- | --- |
| 163 | 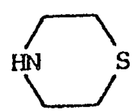 | 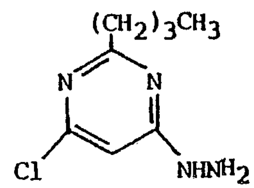 | 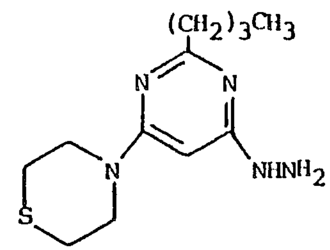 | none taken |
| 164 | 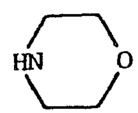 | 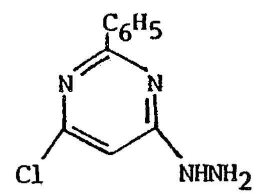 | 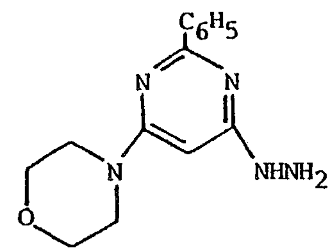 | 140–143 |

Examples 165—171

Using the method of Part F, Example 1, the indicated intermediates of Formula VIII were reacted with the indicated trialkyl orthoesters to provide the indicated compounds of Formula IA (Table XII).

Table XII

| Ex. | Intermediate of Formula VIII | | | Ortho Ester | | Product of Formula IA | | | Calculated: %C; %H; %N<br>Found: %C; %H; %N<br>(m.p. in°C) |
|---|---|---|---|---|---|---|---|---|---|
| | $R_5$ | $R_8$ | X | | $R_3$ | $R_5$ | $R_8$ | X | |
| 165 | $C_6H_5$ | H | O | triethyl orthoformate | H | $C_6H_5$ | H | O | 64.0; 5.4; 24.9<br>63.8; 5.2; 24.7<br>(>280) |
| 166 | H | H | S | trimethyl orthoformate | H | H | H | S | 48.8; 5.0; 31.7<br>48.2; 4.9; 31.3<br>(177-179) |
| 167 | H | H | $NCH_3$ | trimethyl orthoformate | H | H | H | $NCH_3$ | 55.0; 6.5; 38.5<br>54.9; 6.4; 38.1<br>(167-170) |

Table XII (Continued)

| Ex. No. | Intermediate of Formula VIII | | | Ortho Ester | Product of Formula IA | | | | Calculated: %C; %H; %N<br>Found: %C; %H; %N<br>(m.p. in °C) |
|---|---|---|---|---|---|---|---|---|---|
| | $R_5$ | $R_8$ | X | | $R_3$ | $R_5$ | $R_8$ | X | |
| 168 | $CH_3$ | H | $NCH_3$ | triethyl orthoformate | H | $CH_3$ | H | $NCH_3$ | 56.9; 6.9; 36.2<br>56.8; 6.8; 36.6<br>(177–179) |
| 169 | $(CH_2)_2CH_3$ | H | $NCH_3$ | triethyl orthopropionate | $CH_2CH_3$ | $(CH_2)_2CH_3$ | H | $NCH_3$ | 62.5; 8.4; 29.1<br>61.9; 8.5; 29.3<br>(126–129) |
| 170 | H | H | S | triethyl orthopropionate | $CH_2CH_3$ | H | H | S | 53.0; 6.1; 28.1<br>53.1; 6.2; 28.1<br>(125–127) |
| 171 | $(CH_2)_2CH_3$ | H | $NCH_3$ | triethyl orthoformate | H | $(CH_2)_2CH_3$ | H | $NCH_3$ | 60.0; 7.7; 32.2<br>59.5; 7.7; 32.0<br>(162–163) |

Example 172

Preparation of 3-Methyl-5-methylthio-8-phenyl-7-(4-thiomorpholino)-1,2,4-triazolo[4,3-c]pyrimidine according to Scheme IV

Part A Preparation of 4-Chloro-2-methylthio-5-phenyl-6-(4-thiomorpholino)pyrimidine

A mixture of 25 g (0.093 mole) of 4,6-dichloro-2-methylthio-5-phenylpyrimidine and 20 g (0.19 mole) of thiomorpholine in 100 ml of methanol was stirred for 16 hours. The solid was separated by filtration and dried to provide white crystals of crude 4-chloro-2-methylthio-5-phenyl-6-(4-thiomorpholino)pyrimidine, m.p. 110—125°C.

Part B Preparation of 4-Hydrazino-2-methylthio-5-phenyl-6-(4-thiomorpholino)pyrimidine

A mixture of 29 g (0.086 mole) of 4-chloro-2-methylthio-5-phenyl-6-(4-thiomorpholino)pyrimidine and 10.0 g (0.2 mole) of hydrazine hydrate in 200 ml of ethanol was heated at its reflux temperature for 70 hours. The mixture was cooled and the product was separated by filtration. The product was white crystals of 4-hydrazino-2-methylthio-5-phenyl-6-(4-thiomorpholino)pyrimidine, m.p. 170—171°C. The structure was confirmed by infrared and nuclear magnetic resonance spectral analyses.

Part C Preparation of 3-Methyl-5-methylthio-8-phenyl-7-(4-thiomorpholino)-1,2,4-triazolo[4,3-c]-pyrimidine

A mixture of 4.2 g (0.012 mole) of 4-hydrazino-2-methylthio-5-phenyl-6-(4-thiomorpholino)pyrimidine and 40 ml of triethyl orthoacetate was heated at its reflux temperature for about 64 hours, and was then allowed to cool. The solid precipitate was collected by filtration, washed with hexane and dried. Recrystallization from ethyl acetate provided 3.0 g of pale green crystals of 3-methyl-5-methylthio-8-phenyl-7-(4-thiomorpholino)-1,2,4-triazolo[4,3-c]pyrimidine, m.p. 171—172°C. This solid was dissolved in 40 ml of hot ethanol and an equimolar amount of sulfuric acid was added. The mixture was cooled and diethyl ether was added to precipitate yellow crystals of 3-methyl-5-methylthio-8-phenyl-7-(4-thiomorpholino)-1,2,4-triazolo[4,3-c]pyrimidine dihydrogen sulfate, m.p. 193—195°C. The salt was neutralized with ammonium hydroxide to reprecipitate the free base which was again recrystallized from ethyl acetate. Analysis: Calculated for $C_{17}H_{19}N_5S_2$: %C, 57.1; %H, 5.4; %N, 19.6; Found: %C, 56.5; %H, 5.4; %N, 19.7.

Example 173

Preparation of 3-ethyl-5-methylthio-8-phenyl-7-(4-thiomorpholino)-1,2,4-triazolo[4,3-c]pyrimidine

Using the method of Example 172, part C, the intermediate of Example 172, part B, was reacted with triethyl orthopropionate to provide 3-ethyl-5-methylthio-8-phenyl-7-(4-thiomorpholino)-1,2,4-triazolo[4,3-c]pyrimidine dihydrogen sulfate, m.p. 205—207°C. Analysis: Calculated for $C_{18}H_{21}N_5S_2 \cdot H_2SO_4$: %C, 46.0; %H, 4.9; %N, 14.9; Found: %C, 45.9; %H, 5.1; %N, 14.9. The crude free base had a melting point of 152—155°C.

Example 174

Preparation of 2,5-Diethyl-7-[4-(1-dioxothiomorpholino]-1,2,4-triazolo[1,5-c]pyrimidine

To a stirred solution of 5.5 g (20 mmoles) of 2,5-diethyl-7-[4-(thiomorpholino]-1,2,4-triazolo[1,5-c]-pyrimidine in 100 ml of chloroform was added dropwise 8.6 g (50 mmoles) of meta-chloroperbenzoic acid in 100 ml of chloroform over 3.5 hours. The mixture was stirred for an additional 16 hours. Thin layer chromatography showed incomplete reaction. An additional 1.5 g of meta-chloroperbenzoic acid was dissolved in 25 ml of chloroform and the solution was added dropwise over 45 minutes. The mixture was stirred for one additional hour. Thin layer chromatography showed that the reaction had progressed but was still incomplete. An additional 1.5 g of meta-chloroperbenzoic acid was dissolved in 25 ml of chloroform and the solution was added dropwise over 45 minutes. After stirring the mixture for about 90 hours it was washed thrice with 10% aqueous sodium hydroxide solution, once with water and once with saturated sodium chloride solution, and was finally dried over magnesium sulfate. Evaporation provided a white solid which was recrystallized from ethyl acetate. The solid product was separated by filtration and found by thin layer chromatography to be primarily the desired product. A second crop was obtained by adding cyclohexane to the ethyl acetate filtrate. The white solid was collected by filtration and determined by thin layer chromatography to be pure 2,5-diethyl-7-[4-(1-dioxothiomorpholino]-1,2,4-triazolo[1,5-c]pyrimidine, m.p. 187—189°C. Analysis: Calculated for $C_{13}H_{19}N_5O_2S$: %C, 50.5; %H, 6.2; %N, 22.6; Found: %C, 50.3; %H, 6.1; %N, 23.0.

To a warm solution of 1.0 g (3.2 mmole) of 2,5-diethyl-7-[4-(1-dioxothiomorpholino]-1,2,4-triazolo[1,5-c]pyrimidine in 50 ml of ethanol was added dropwise 0.30 g (3.1 mmole) of concentrated sulfuric acid. The solution was stirred and allowed to cool to about 20°C, then diluted to a total volume of 300 ml with diethyl ether. After stirring for one hour the solid was collected by filtration, rinsed with ether and dried to provide 2,5-diethyl-7-[4-(1-dioxothiomorpholino]-1,2,4-triazolo[1,5-c]pyrimidine bisulfate, m.p. 189—191°C. Analysis: Calculated for $C_{13}H_{19}N_5O_2S \cdot H_2SO_4$: %C, 38.3; %H, 5.2; %N, 17.2; Found %C, 38.5; %H, 5.2; %N, 17.6

Example 175

Preparation of 2,5-Diethyl-7-[4-(1-oxothiomorpholino]-1,2,4-triazolo[1,5-c]pyrimidine

To a stirred solution of 8.3 g (30 mmoles) of 2,5-diethyl-7-[4-thiomorpholino]-1,2,4-triazolo[1,5-c]-pyrimidine in 75 ml of chloroform was added, dropwise over 35 minutes, 80 ml of a chloroform solution of 5.2 g (30 mmoles) of meta-chloroperbenzoic acid. A mild exotherm was observed, and the solution was stirred for an additional 45 minutes. Thin layer chromatography showed incomplete reaction. An additional 0.5 g of meta-chloroperbenzoic acid was dissolved in 10 ml of chloroform and the solution was added dropwise to the reaction mixture. After stirring for an additional 1.5 hours the reaction mixture was washed sequentially with 10% sodium hydroxide solution, water and saturated sodium chloride solution, and was then dried over magnesium sulfate. The dried organic layer was then evaporated to provide an off-white solid residue. Chromatography on silica gel, eluting with 1:9 methanol:chloroform provided starting material in early fractions and product in later fractions. The product was white solid 2,5-diethyl-7-[4-(1-di-oxothiomorpholino]-1,2,4-triazolo[1,5-c]pyrimidine m.p. 223—225°C. The structure was confirmed by $C^{13}$ nuclear magnetic resonance and infrared spectral analyses. Analysis: Calculated for $C_{13}H_{19}N_5OS$: %C, 53.2; %H, 6.5; %N, 23.9; Found: %C, 53.1; %H, 6.4; %N, 23.8.

To a warm solution of 2.5 g (8.5 mmoles) of 2,5-diethyl-7-[4-(1-oxothiomorpholino]-1,2,4-triazolo[1,5-c]pyrimidine in 60 ml of ethanmol was added dropwise 0.82 g (8.4 mmole) of concentrated sulfuric acid. The solution was allowed to cool to about 20°C, then diluted to a total volume of 250 ml by the addition of diethyl ether. The mixture was stirred for one hour, and the solid was then separated by filtration. The solid was washed with ether and dried to provide wite solid 2,5-diethyl-7-[4-(1-oxothiomorpholino]-1,2,4-triazolo[1,5-c]pyrimidine bisulfate, m.p. 210—212°C. Analysis: Calculated for $C_{13}H_{19}N_5OS \cdot H_2SO_4$: %C, 39.98; %H, 5.4; %N, 17.9; Found: %C, 39.9; %H, 5.4; %N, 18.2

**Claims for the Contracting States: BE CH DE FR GB IT LI NL SE**

1. A compound of the formula:

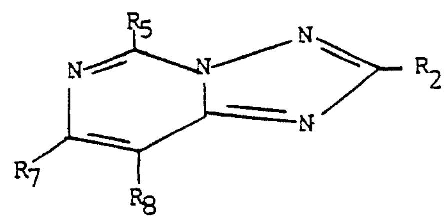

IB

or a pharmaceutically acceptable salt thereof, wherein $R_2$ is a hydrogen or alkyl of 1 to 4 carbon atoms; at least one of $R_5$ and $R_2$ if

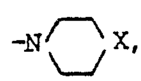

wherein the or each X is independently oxygen, sulphur, sulphinyl, sulphonyl, methylene, imino or N-alkylimino of 1 to 4 carbon atoms, the other of $R_5$ and $R_7$, if not

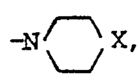

being hydrogen, alkyl of 1 to 4 carbon atoms, or phenyl; and $R_8$ is hydrogen, alkyl of 1 to 4 carbon atoms or phenyl; with the proviso that if $R_5$ is

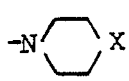

and $R_7$ is methyl, then X is sulphinyl or sulphonyl.

2. A compound of pharmaceutically acceptable salt thereof according to claim 1, wherein X is sulphur or oxygen.

3. A compound or pharmaceutically acceptable salt thereof according to claim 1 or claim 2, wherein $R_5$ is hydrogen or alkyl of 1 to 4 carbon atoms.

4. A compound or pharmaceutically acceptable salt thereof according to any proceeding claim wherein $R_8$ is hydrogen.

5. A compound according to claim 4 which is:
5,7-bis(4-morpholino)-1,2,4-triazolo[1,5-c]pyrimidine,
5-(1-morpholino)-7-(4-thiomorpholino)-1,2,4-triazolo[1,5-c]pyrimidine,
2-ethyl-5-methyl-7-(4-thiomorpholino)-1,2,4-triazolo[1,5-c]pyrimidine,
2-ethyl-5-methyl-7-(4-morpholino)-1,2,4-triazolo[1,5-c]pyrimidine,
2,5-diethyl-7-(4-thiomorpholino)-1,2,4-triazolo[1,5-c]pyrimidine,
2-ethyl-7-(4-morpholino)-5-*n*-propyl-1,2,4-triazolo[1,5-c]pyrimidine,

7-(4-morpholino)-5-*n*-propyl-1,2,4-triazolo[1,5-c]pyrimidine,
2-ethyl-5-*n*-propyl-7-(4-thiomorpholino)-1,2,4-triazolo[1,5-c]pyrimidine,
5-*n*-propyl-7-(4-thiomorpholino)-1,2,4-triazolo[1,5-c]pyrimidine,
5-ethyl-7-(4-morpholino)-1,2,4-triazolo[1,5-c]pyrimidine,
5-ethyl-2-methyl-7-(4-morpholino)-1,2,4-triazolo[1,5-c]pyrimidine,
2,5-diethyl-7-(4-morpholino)-1,2,4-triazolo[1,5-c]pyrimidine.

6. A process for preparing a triazolo[1,5-c]pyrimidine of the formula:

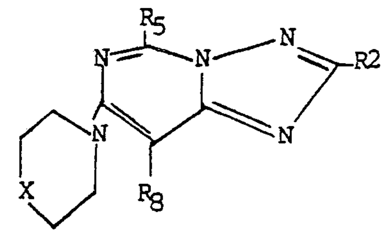

wherein $R_2$ is hydrogen or alkyl of 1 to 4 carbon atoms, $R_5$ is hydrogen, alkyl of 1 to 4 carbon atoms or phenyl; $R_8$ is hydrogen, alkyl of 1 to 4 carbon atoms or phenyl; and X is oxygen, sulphur, sulphinyl, sulphonyl, methylene, imino or N-alkylimino of 1 to 4 carbon atoms comprising heating a triazolo[4,3-c]pyrimidine of the formula:

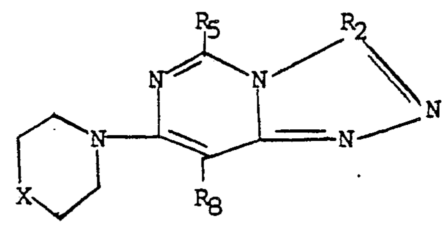

$R_2$, $R_5$, $R_8$ and X being as defined above, in the presence of an aqueous acid to provide said triazolo-[1,5-c]pyrimidine.

7. A process as claimed in claim 6 wherein said triazolo[4,3-c]pyrimidine is obtained by heating a compound of the formula:

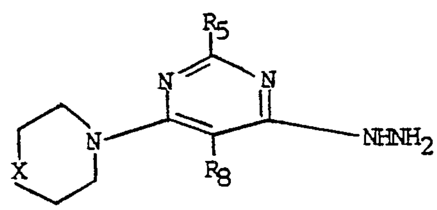

$R_5$, $R_8$ and X being as defined in claim 6, in the presence of a trialkyl orthoester of the formula:

$$R_2C(OAlk)_3$$

wherein $R_2$ is as defined in claim 6 and Alk is alkyl of 1 to 4 carbon atoms.

8. A process for preparing a triazolo[1,5-c]pyrimidine of the formula:

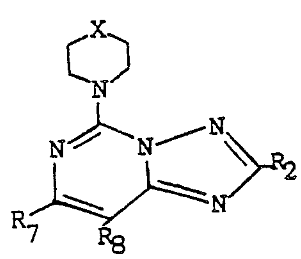

wherein $R_2$ and $R_7$ are each hydrogen or alkyl of 1 to 4 carbon atoms and $R_8$ and X are as defined in claim 1, comprising heating a triazolo[4,3-c]pyrimidine of the formula:

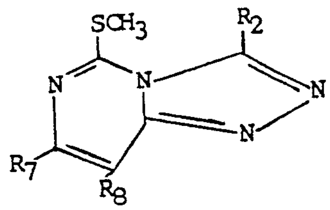

$R_2$, $R_7$ and $R_8$ being as defined above, in the presence of an excess of heterocyclic amine of formula:

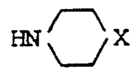

X being as defined above.

9. A compound or a pharmaceutically acceptable salt thereof as claimed in any of claims 1 to 5 when produced by a method as claimed in any of claims 6, 7 and 8.

10. A compound of the formula:

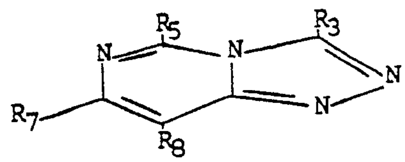

IA

wherein $R_3$ is hydrogen or alkyl of 1 to 4 carbon atoms; one of $R_5$ and $R_7$ is the heterocyclic substituent

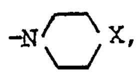

wherein X is oxygen, sulfur, sulfinyl, sulfonyl, methylene, imino or N-alkylimino of 1 to 4 carbon atoms when $R_5$ is said heterocyclic substituent, $R_7$ is hydrogen or alkyl of 1 to 4 carbon atoms; when $R_7$ is said heterocyclic substituent, $R_5$ is hydrogen, alkyl or alkylthio of 1 to 4 carbon atoms or phenyl; and $R_8$ is hydrogen, phenyl or alkyl of 1 to 4 carbon atoms; or a pharmaceutically acceptable salt thereof.

11. A compound according to claim 10 which is:
3,5-bis(n-propyl)-7-(4-thiomorpholino)-1,2,4-triazolo[4,3-c]pyrimidine,
5-ethyl-3-methyl-7-(4-thiomorpholino)-1,2,4-triazolo[4,3-c]pyrimidine,
3-methyl-5-methylthio-8-phenyl-7-(4-thiomorpholino)-1,2,4-triazolo[4,3-c]pyrimidine,
5-ethyl-3-isopropyl-7-(4-thiomorpholino)-1,2,4-triazolo[4,3-c]pyrimidine,
3-ethyl-5-methyl-7-(4-thiomorpholino)-1,2,4-triazolo[4,3-c]pyrimidine,
3,5-bis(n-propyl)-7-(4-morpholino)-1,2,4-triazolo[4,3-c]pyrimidine,
3,5-diethyl-7-(1-piperidino)-1,2,4-triazolo[4,3-c]pyrimidine,
7-(4-morpholino)-5-methylthio-1,2,4-triazolo[4,3-c]pyrimidine,
3-ethyl-5-*n*-propyl-7-(4-thiomorpholino)-1,2,4-triazolo[4,3-c]pyrimidine,
7-(4-methyl-1-piperazino)-5-(n-propyl)-1,2,4-triazolo[4,3-c]pyrimidine,
5-*n*-propyl-7-(4-thiomorpholino)-1,2,4-triazolo[4,3-c]pyrimidine,
5-ethyl-7-(4-thiomorpholino)-1,2,4-triazolo[4,3-c]pyrimidine,
5-ethyl-3-methyl-7-(4-thiomorpholino)-1,2,4-triazolo[4,3-c]pyrimidine, or
3,5-diethyl-7-(4-thiomorpholino)-1,2,4-triazolo[4,3-c]pyrimidine.

12. A pharmaceutical composition comprising a compound or pharmaceutically acceptable salt thereof as claimed in any of claims 1 to 5 or 9 to 11 and a pharmaceutically acceptable carrier.

13. A pharmaceutical composition as claimed in claim 12 in the form of a tablet, capsule, suspension, solution or suppository, said compound or pharmaceutically acceptable salt thereof being present in an amount sufficient to induce bronchodilation in a mammal.

14. Use of a compound or pharmaceutically acceptable salt thereof either as claimed in any of claims 1 to 5 or 9 to 11, or as defined in claim 1 but without said proviso which relates to $R_5$, $R_7$ and X, for the preparation of a medicament for inducing bronchodilation in a mammal.

**Claims for the Contracting State: AT**

1. A process for preparing a triazolo[1,5-c]pyrimidine of the formula:

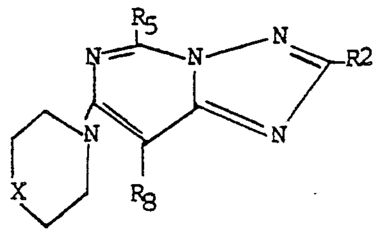

wherein $R_2$ is hydrogen or alkyl of 1 to 4 carbon atoms, $R_5$ is hydrogen, alkyl of 1 to 4 carbon atoms or phenyl; $R_8$ is hydrogen, alkyl of 1 to 4 carbon atoms or phenyl; and X is oxygen, sulphur, sulphinyl, sulphonyl, methylene, imino or N-alkylimino of 1 to 4 carbon atoms comprising heating a triazolo-[4,3-c]pyrimidine of the formula:

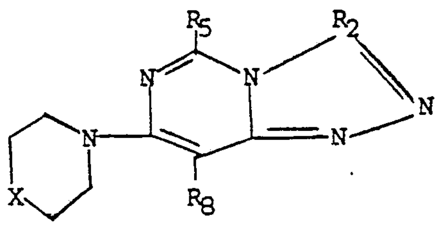

$R_2$, $R_5$, $R_8$ and X being as defined above, in the presence of an aqueous acid to provide said triazolo-[1,5-c]pyrimidine.

2. A process as claimed in claim 1 wherein said triazolo[4,3-c]pyrimidine is obtained by heating a compound of the formula:

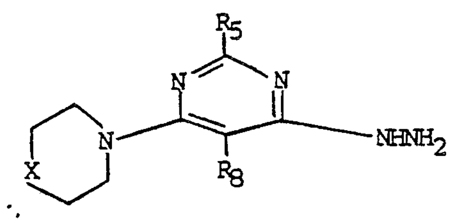

$R_5$, $R_8$ and X being as defined in claim 1, in the presence of a trialkyl orthoester of the formula:

$$R_2C(OAlk)_3$$

wherein $R_2$ is as defined in claim 1 and Alk is alkyl of 1 to 4 carbon atoms.

3. A process for preparing a triazolo[1,5-c]pyrimidine of the formula:

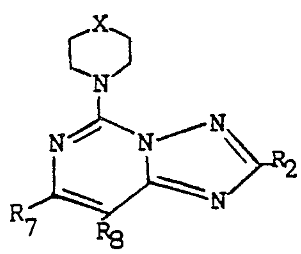

wherein $R_2$ and $R_7$ are each hydrogen or alkyl of 1 to 4 carbon atoms and $R_8$ and X are as defined in claim 1, comprising heating a triazolo[4,3-c]pyrimidine of the formula:

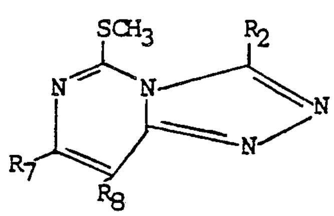

$R_2$, $R_7$ and $R_8$ being as defined above, in the presence of an excess of heterocyclic amine of formula:

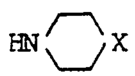

X being as defined above.

4. A process for the manufacture of a medicament comprising preparing a triazolo pyrimidine by a method as claimed in any of claims 1 to 3 and bringing the resulting triazolo pyrimidine into association with a pharmaceutically acceptable vehicle.

5. Use of a process as claimed in any of claims 1 to 3 for the manufacture of a medicament for inducing bronchodilation in a mammal.

**Patentansprüche für die Vertragsstaaten: BE CH DE FR GB IT LI NL SE**

1. Verbindung der Formel

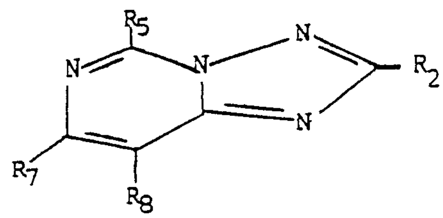

IB

oder pharmazeutisch verwendbares Salz derselben, in der $R_2$ Wasserstoff oder ein Alkyl mit 1 bis 4 Kohlenstoffatomen ist; mindestens einer der Anteile $R_5$ und $R_7$

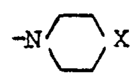

ist, wobei das oder jedes X unabhängig Sauerstoff, Schwefel, Sulfinyl, Sulfonyl, Methylen oder Imino oder N-Alkylamino mit 1 bis 4 Kohlenstoffatomen ist, der andere der Anteile $R_5$ udn $R_7$, wenn er nicht

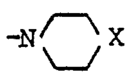

ist, Wasserstoff oder ein Alkyl mit 1 bis 4 Kohlenstoffatomen oder ein Phenyl ist, und, wenn $R_5$

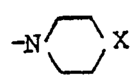

ist und $R_7$ Methyl ist, X Sulfinyl oder Sulfonyl ist.

2. Verbindung oder pharmazeutisch verwendbares Salz derselben nach Anspruach 1, dadurch gekennzeichnet, daß X Schwefel oder Sauerstoff ist.

3. Verbindung oder pharmazeutisch verwendbares Salz derselben nach Anspruch 1 oder 2, dadurch gekennzeichnet, daß $R_5$ Wasserstoff oder ein Alkyl mit 1 bis 4 Kohlenstoffatomen ist.

4. Verbindung oder pharmazeutisch verwendbares Salz derselben nach einem der vorhergehenden Ansprüche, dadurch gekennzeichnet, daß $R_8$ Wasserstoff ist.

5. Verbindung nach Anspruch 4, die
5,7-Bis(4-morpholino)-1,2,4-triazolo[1,5-c]pyrimidin,
5-(1-Morpholino)-7-(4-thiomorpholino)-1,2,4-triazolo[1,5-c]pyrimidin,
2-Ethyl-5-methyl-7-(4-thiomorpholino)-1,2,4-triazolo[1,5-c]pyrimidin,
2-Ethyl-5-methyl-7-(4-morpholino)-1,2,4-triazolo[1,5-c]pyrimidin,
2,5-Diethyl-7-(4-thiomorpholino)-1,2,4-triazolo[1,5-c]pyrimidin,
2-Ethyl-7-(4-morpholino)-5-*n*-propyl-1,2,4-triazolo[1,5-c]pyrimidin,
7-(4-Morpholino)-5-*n*-propyl-1,2,4-triazolo[1,5-c]pyrimidin,
2-Ethyl-5-*n*-propyl-7-(4-thiomorpholino)-1,2,4-triazolo[1,5-c]pyrimidin,
5-*n*-Propyl-7-(4-thiomorpholino)-1,2,4-triazolo[1,5-c]pyrimidin,
5-Ethyl-7-(4-morpholino)-1,2,4-triazolo[1,5-c]pyrimidin,
5-Ethyl-2-methyl-7-(4-morpholino)-1,2,4-triazolo[1,5-c]pyrimidin, oder
2,5-Diethyl-7-(4-morpholino)-1,2,4-triazolo[1,5-c]pyrimidine ist.

6. Verfahren zum Erzeugen eines Triazolo[1,5-c]pyrimidins der Formel

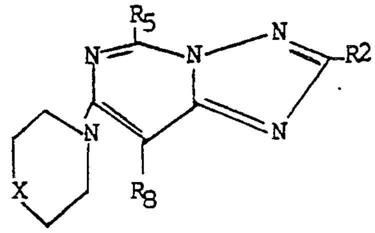

in der $R_2$ Wasserstoff oder ein Alkyl mit 1 bis 4 Kohlenstoffatomen ist, $R_5$ Wasserstoff, ein Alkyl mit 1 bis 4 Kohlenstoffatomen oder ein Phenyl ist; $R_8$ Wasserstoff, ein Alkyl mit 1 bis 4 Kohlenstoffatomen oder ein Phenyl ist, und X Sauerstoff, Schwefel, Sulfinyl, Sulfonyl, Methylen, ein Imino oder N-Alkylimino mit 1 bis 4 Kohlenstoffatomen ist, in dem ein Triazolo[4,3-c]pyrimidin der Formel

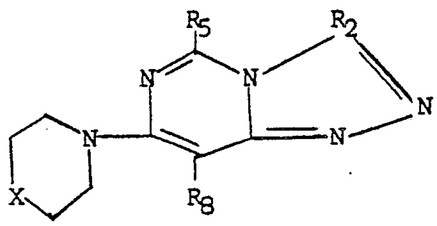

in der $R_2$, $R_5$, $R_8$ und X die vorstehend angegebenen Bedeutungen haben, zur Bildung des genannten Triazolo[1,5-c]Pyrimidins in Gegenwart einer wässrigen Säure erhitzt wird.

7. Verfahren nach Anspruch 6, dadurch gekennzeichnet, daß das Triazolo[4,3-c]Pyrimidin erzeugt wird, indem eine Verbindung der Formel

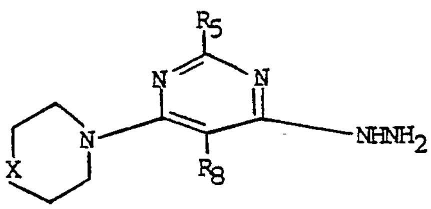

in der $R_5$, $R_8$ und X die im Anspruch 6 angegebenen Bedeutungen haben, in Gegenwart eines Trialkylorthoesters der Formel

$$R_2C(OAlk)_3$$

erhitzt werden, in der $R_2$ die im Anspruch 6 angegebene Bedeutung hat und Alk ein Alkyl mit 1 bis 4 Kohlenstoffatomen ist.

8. Verfahren zum Erzeugen eines Triazolo[1,5-c]pyrimidins der Formel

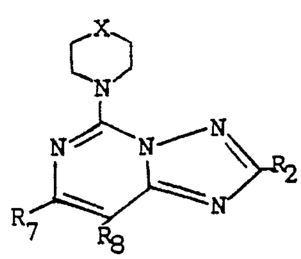

in der $R_2$ und $R_7$ jeweils Wasserstoff oder ein Alkyl mit 1 bis 4 Kohlenstoffatomen sind und $R_8$ und X die im Anspruch 1 angegebenen Bedeutungen haben, in dem ein Triazolo[4,3-c]pyrimidin der Formel

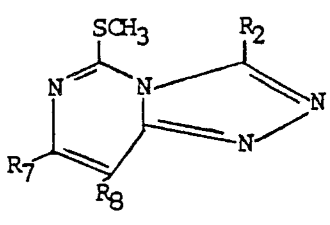

in der $R_2$, $R_7$ und $R_8$ die vorstehend angegebenen Bedeutungen haben, in Gegenwart eines überschusses eines heterozyklischen Amins der Formel

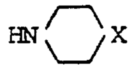

erhitzt wird, in der X die vorstehend angegebene Bedeutung hat.

9. Verbindung oder pharmazeutisch verwendbares Salz derselben nach einem der Ansprüche 1 bis 5, sofern sie bzw. es nach einem Verfahren nach eines der Ansprüche 6, 7 und 8 erzeugt worden ist.

10. Verbindung der Formel

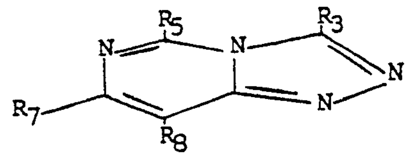

in der $R_3$ Wasserstoff oder ein Alkyl mit 1 bis 4 Kohlenstoffatomen ist, einer der Anteile $R_5$ und $R_7$ der heterozyklische Substituent ist, ind dem X Sauerstoff, Schwefel, Sulfinyl, Sulfonyl, Methylen, Imino oder N-Alkylamino mit 1 bis 4 Kohlenstoffatomen ist, wenn $R_5$ der heterozyklische Substituent ist, $R_7$ Wasserstoff oder ein Alkyl mit 1 bis 4 Kohlenstoffatomen ist, wenn $R_7$ der heterozyklische Substituent ist, ferner $R_5$ Wasserstoff oder ein Alkyl oder Alkylthio mit 1 bis 4 Kohlenstoffatomen oder ein Phenyl ist und $R_8$ Wasserstoff oder ein Phenyl oder ein Alkyl mit 1 bis 4 Kohlenstoffatomen ist; oder ein pharmazeutisch verwendbares Salz der Verbindung.

11. Verbindung nach Anspruch 10, die
3,5-Bis(n-propyl)-7-(4-thiomorpholino)-1,2,4-triazolo[4,3-c]pyrimidin,
5-Ethyl-3-methyl-7-(4-thiomorpholino)-1,2,4-triazolo[4,3-c]pyrimidin,
3-Methyl-5-methylthio-8-phenyl-7-(4-thiomorpholino)-1,2,4-triazolo[4,3-c)pyrimidin,
5-Ethyl-3-isopropyl-7-(4-thiomorpholino)-1,2,4-triazolo[4,3-c]pyrimidin,
3-Ethyl-5-methyl-7-(4-thiomorpholino)-1,2,4-triazolo[4,3-c]pyrimidin,
3,5-Bis(n-propyl)-7-(4-thiomorpholino)-1,2,4-triazolo[4,3-c]pyrimidin,
3,5-Diethyl-7-(1-piperidino)-1,2,4-triazolo[4,3-c]pyrimidin,
7-(4-Morpholino)-5-methylthio-1,2,4-triazolo[4,3-c]pyrimidin,
3-Ethyl-5-*n*-propyl-7-(4-thiomorpholino)-1,2,4-triazolo[4,3-c]pyrimidin,
7-(4-Methyl-1-piperazino)-5-(n-propyl)-1,2,4-triazolo[4,3-c]pyrimidin,
5-*n*-Propyl-7-(4-thiomorpholino)-1,2,4-triazolo[4,3-c]pyrimidin,
5-Ethyl-7-(4-thiomorpholino)-1,2,4-triazolo[4,3-c]pyrimidin,
5-Ethyl-3-methyl-7-(4-morpholino)-1,2,4-triazolo[4,3-c]pyrimidin, oder
3,5-Diethyl-7-(4-thiomorpholino)-1,2,4-triazolo[4,3-c]pyrimidin.

12. Pharmazeutisch Zusammensetzung mit einer Verbindung oder einem pharmazeutisch verwendbaren Salz derselben nach einem der Ansprüche 1 bis 5 oder 9 bis 11 und mit einem pharmazeutisch verwendbaren Träger.

13. Pharmazeutische Zusammensetzung nach Anspruch 12 in Form einer Tablette, Kapsel, Suspension oder Lösung oder eines Zäpfchens, wobei die Verbindung oder deren pharmazeutisch verwendbares Salz in einer Menge vorhanden ist, die zum Herbeiführen einer Bronchienerweiterung bei einem Säugetier genügt.

14. Verwendung einer Verbindung oder eines pharmazeutisch verwendbaren Salzes derselben nach einem der Ansprüche 1 bis 5 oder 9 bis 11 oder nach Anspruch 1, jedoch ohne die $R_5$, $R_7$ und betrefende Bestimmung, zur Herstellung eines Medikaments zum Herbeiführen einer Bronchienerweiterung bei einem Säugetier.

**Patentansprüche für den Vertragsstaat: AT**

1. Verfahren zum Erzeugen eines Triazolo[1,5-c]pyrimidins der Formel

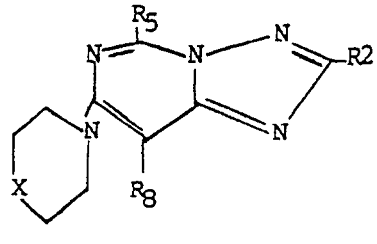

in der $R_2$ Wasserstoff oder ein Alkyl mit 1 bis 4 Kohlenstoffatomen ist, $R_5$ Wasserstoff, ein Alkyl mit 1 bis 4

Kohlenstoffatomen oder ein Phenyl ist; $R_8$ Wasserstoff, ein Alkyl mit 1 bis 4 Kohlenstoffatomen oder ein Phenyl ist, und X Sauerstoff, Schwefel, Sulfinyl, Sulfonyl, Methylen, ein Imino oder N-Alkylimino mit 1 bis 4 Kohlenstoffatomen ist, in dem ein Triazolo[4,3-c]pyrimidin der Formel

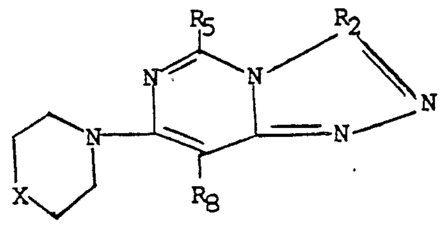

in der $R_2$, $R_5$, $R_8$ und X die vorstehend angegebenen Bedeutungen haben, zur Bildung des genannten Triazolo[1,5-c]Pyrimidins in Gegenwart einer wässrigen Säure erhitzt wird.

2. Verfahren nach Anspruch 1, dadurch gekennzeichnet, daß das Triazolo[4,3-c]Pyrimidin erzeugt wird, indem eine Verbindung der Formel

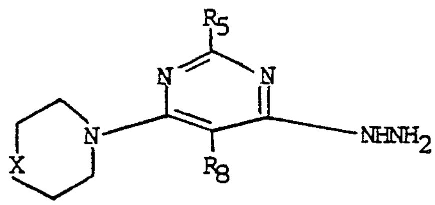

in der $R_5$, $R_8$ und X die im Anspruch 1 angegebenen Bedeutungen haben, in Gegenwart eines Trialkylorthoesters der Formel

$$R_2C(OAlk)_3$$

erhitzt werden, in der $R_2$ die im Anspruch 1 angegebene Bedeutung hat und Alk ein Alkyl mit 1 bis 4 Kohlenstoffatomen ist.

3. Verfahren zum Erzeugen eines Triazolo[1,5-c]pyrimidins der Formel

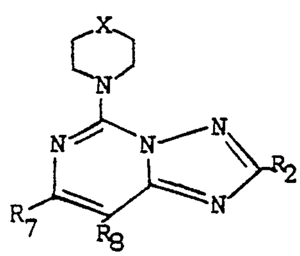

in der $R_2$ und $R_7$ jeweils Wasserstoff oder ein Alkyl mit 1 bis 4 Kohlenstoffatomen sind und $R_8$ und X die im Anspruch 1 angegebenen Bedeutungen haben, in dem ein Triazolo[4,3-c]pyrimidin der Formel

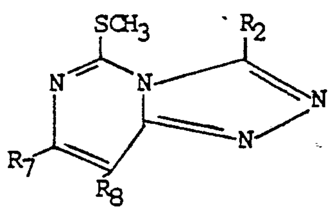

in der $R_2$, $R_7$ und $R_8$ vorstehend angegebenen Bedeutungen haben, in Gegenwart eines überschusses eines heterozyklischen Amins der Formel

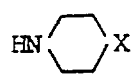

erhitzt wird, in der X die vorstehend angegebene Bedeutung hat.

4. Verfahren zum Herstellen eines Medikaments, in dem nach einem Verfahren nach einem der Ansprüche 1 bis 3 ein Triazolopyrimidin erzeugt und dieses einem pharmazeutisch verwendbaren Träger zugeordnet wird.

5. Die Anwendung eines Verfahrens nach einem der Ansprüche 1 bis 7 zur Herstellung eines Medikaments zum Herbeiführen einer Bronchienerweiterung bei einem Säugetier.

**Revendications pour les Etats contractants: BE CH DE FR GB IT LI NL SE**

1. Composé répondant à la formule:

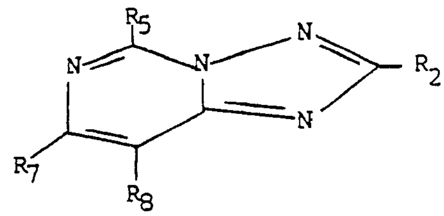

IB

ou un sel acceptable en pharmacie d'un tel composé, formule dans laquelle $R_2$ est l'hydrogène ou un radical alkyle de 1 à 4 atomes de carbone; au moins l'un des symboles $R_5$ et $R_7$ représente le groupe

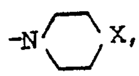

où le ou chaque X représente indépendamment de l'oxygène, du soufre, le radical sulfinyle, le radical sulfonyle, le radical méthylène, le radical imino ou le radical N-alkylimino de 1 à 4 atomes de carbone, l'autre des symboles $R_5$ et $R_7$, s'il ne représente pas le groupe

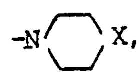

étant de l'hydrogène, un radical alkyle de 1 à 4 atomes de carbone ou le radical phényle et $R_8$ représente l'hydrogène, un radical alkyle de 1 à 4 atomes de carbone ou le radical phényle; à la condition que, si $R_5$ représente le groupe;

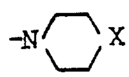

et si $R_7$ est le radical méthyle, X représente alors un radical sulfinyle ou sulfonyle.

2. Composé ou sel acceptable en pharmacie de celui-ci suivant la revendication 1, où X représente le soufre ou l'oxygène.

3. Composé ou sel acceptable en pharmacie de celui-ci suivant la revendication 1 ou la revendication 2, où $R_5$ est l'hydrogène ou un radical alkyle de 1 à 4 atomes de carbone.

4. Composé ou sel acceptable en pharmacie de celui-ci suivant l'une quelconque des revendications précédentes, où $R_8$ représente l'hydrogène.

5. Composé suivant la revendication 4, qui est:
la 5,7-bis(4-morpholino)-1,2,4-triazolo[1,5-c]pyrimidine,
la 5-(1-morpholino)-7-(4-thiomorpholino)-1,2,4-triazolo[1,5-c]pyrimidine,
la 2-éthyl-5-méthyl-7-(4-thiomorpholino)-1,2,4-triazolo[1,5-c]pyrimidine,
la 2-éthyl-5-méthyl-7-(4-morpholino)-1,2,4-triazolo[1,5-c]pyrimidine,
la 2,5-diéthyl-7-(4-thiomorpholino)-1,2,4-triazolo[1,5-c]pyrimidine,
la 2-éthyl-7-(4-morpholino)-5-*n*-propyl-1,2,4-triazolo[1,5-c]pyrimidine,
la 7-(4-morpholino)-5-*n*-propyl-1,2,4-triazolo[1,5-c]pyrimidine,
la 2-éthyl-5-*n*-propyl-7-(4-thiomorpholino)-1,2,4-triazolo[1,5-c]pyrimidine,
la 5-*n*-propyl-7-(4-thiomorpholino)-1,2,4-triazolo[1,5-c]pyrimidine,
la 5-éthyl-7-(4-morpholino)-1,2,4-triazolo[1,5-c]pyrimidine,
la 5-éthyl-2-méthyl-7-(4-morpholino)-1,2,4-triazolo[1,5-c]pyrimidine,
la 2,5-diéthyl-7-(4-morpholino)-1,2,4-triazolo[1,5-c]pyrimidine.

6. Procédé de préparation d'une triazolo[1,5-c]pyrimidine répondant à la formule:

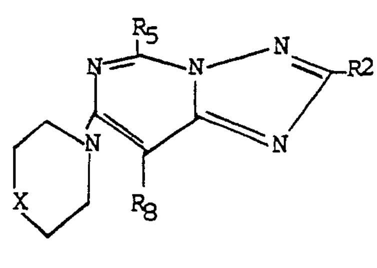

dans laquelle $R_2$ représente l'hydrogène ou un radical alkyle de 1 à 4 atomes de carbone; $R_5$ représente l'hydrogène, un radical alkyle de 1 à 4 atomes de carbone ou le radical phényle; $R_8$ représente l'hydrogène, un radical alkyle de 1 à 4 atomes de carbone ou le radical phényle; et X représente l'oxygène, le soufre, le radical sulfinyle, le radical sulfonyle, le radical méthylène, un radical imino ou un radical N-alkylimino de 1 à 4 atomes de carbone, comprenant le chauffage d'une triazolo[4,3-c]pyrimidine répondant à la formule:

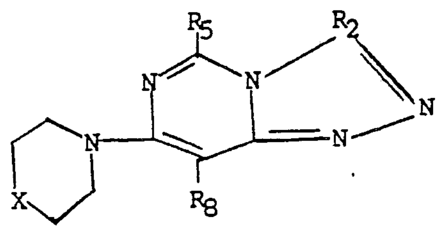

dans laquelle $R_2$, $R_5$, $R_8$ et X ont la définition donnée précédemment, en présence d'un acide aqueux pour former la triazolo[1,5-c]pyrimidine susdite.

7. Procédé suivant la revendication 6, caractérisé en ce que la triazolo[4,3-c]pyrimidine s'obtient par chauffage d'un composé de la formule:

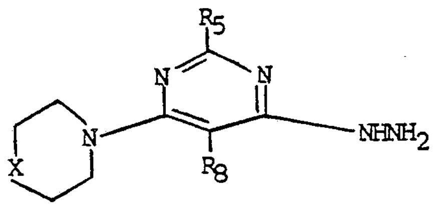

dans laquelle $R_5$, $R_8$ et X ont la définition donnée dans la revendication 6, en présence d'un orthoester de trialkyle de la formule:

$$R_2C(OAlk)_3$$

où $R_2$ a la définition donnée dans la revendication 6 et Alk représente un radical alkyle de 1 à 4 atomes de carbone.

8. Procédé de préparation d'une triazolo[1,5-c]pyrimidine de la formule:

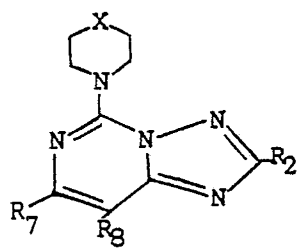

dans laquelle $R_2$ et $R_7$ représentent chacun de l'hydrogène ou un radical alkyle de 1 à 4 atomes de carbone, et $R_8$ et X ont la définition donnée dans la revendication 1, comprenant le chauffage d'une triazolo-[4,3-c]pyrimidine de la formule:

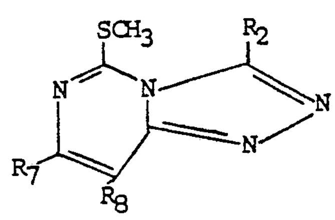

dans laquelle $R_2$, $R_7$ et $R_8$ ont la définition donnée précédemment, en présence d'un excès d'une amine hétérocyclique de la formule:

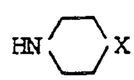

où X a la définition donnée précédemment.

9. Composé ou sel acceptable en pharmacie d'un tel composé suivant l'une quelconque des revendications 1 à 5, lorsqu'il est produit par un procédé suivant l'une quelconque des revendication 6, 7 et 8.

10. Composé répondant à la formule:

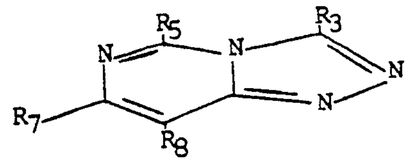

IA

dans laquelle $R_3$ représente l'hydrogène ou un radical alkyle de 1 à 4 atomes de carbone; l'un des symboles $R_5$ et $R_7$ représente le substituant hétérocyclique

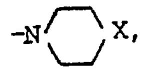

où X représente l'oxygène, le soufre, le radical sulfinyle, le radical sulfonyle, le radical méthylène, un radical imino ou un radical N-alklyimino de 1 à 4 atomes de carbone; lorsque $R_5$ est le substituant hétérocyclique, $R_7$ représente l'hydrogène ou un radical alkyle de 1 à 4 atomes de carbone; lorsque $R_7$ est le substituant hétérocyclique, $R_5$ représente l'hydrogène, un radical alkyle ou un radical alkylthio de 1 à 4 atomes de carbone ou le radical phényle; et $R_8$ représente l'hydrogène, le radical phényle ou un radical alkyle de 1 à 4 atomes de carbone; ou un sel acceptable en pharmacie d'un tel composé.

11. Composé suivant la revendication 10, qui est:
la 3,5-bis(n-propyl)-7-(4-thiomorpholino)-1,2,4-triazolo[4,3-c]pyrimidine,
la 5-éthyl-3-méthyl-7-(4-thiomorpholino)-1,2,4-triazolo[4,3-c]pyrimidine,
la 3-méthyl-5-méthylthio-8-phényl-7-(4-thiomorpholino)-1,2,4-triazolo[4,3-c]pyrimidine,
la 5-éthyl-3-isopropyl-7-(4-thiomorpholino)-1,2,4-triazolo[4,3-c]pyrimidine,
la 3-éthyl-5-méthyl-7-(4-thiomorpholino)-1,2,4-triazolo[4,3-c]pyrimidine,
la 3,5-bis(n-propyl)-7-(4-thiomorpholino)-1,2,4-triazolo[4,3-c]pyrimidine,
la 3,5-diéthyl-7-(1-pipéridino)-1,2,4-triazolo[4,3-c]pyrimidine,
la 7-(4-morpholino)-5-méthylthio-1,2,4-triazolo[4,3-c]pyrimidine,
la 3-éthyl-5-*n*-propyl-7-(4-thiomorpholino)-1,2,4-triazolo[4,3-c]pyrimidine,
la 7-(4-méthyl-1-piperazino)-5-(n-propyl)-1,2,4-triazolo[4,3-c]pyrimidine,
la 5-*n*-propyl-7-(4-thiomorpholino)-1,2,4-triazolo[4,3-c]pyrimidine,
la 5-éthyl-7-(4-morpholino)-1,2,4-triazolo[4,3-c]pyrimidine,
la 5-éthyl-3-méthyl-7-(4-thiomorpholino)-1,2,4-triazolo[4,3-c]pyrimidine, ou
la 3,5-diéthyl-7-(4-thiomorpholino)-1,2,4-triazolo[4,3-c]pyrimidine.

12. Composition pharmaceutrique comprenant un composé ou un sel acceptable en pharmacie de celui-ci suivant l'une quelconque des revendications 1 à 5 ou 9 à 11 et un véhicule acceptable en pharmacie.

13. Composition pharmaceutique suivant la revendication 12, se présentant sous la forme d'un comprimé, d'une gélule, d'une suspension, d'une solution ou d'un suppositoire, le composé susdit ou un sel acceptable en pharmacie de celui-ci étant présent en une quantité suffisante pour provoquer une bronchodilatation chez les mammifères.

14. Utilisation d'un composé ou d'un sel acceptable en pharmacie de celui-ci, suivant l'une quelconque des revendications 1 à 5 ou 9 à 11, ou tel que défini dans la revendication 1 mais sans la condition qui se rapporte à $R_5$, $R_7$ et X, dans la préparation d'un médicament destiné à provoquer une bronchodilatation chez les mammifères.

**Revendications pour l'Etat contractant: AT**

1. Procédé de préparation d'une triazolo[1,5-c]pyrimidine de la formule:

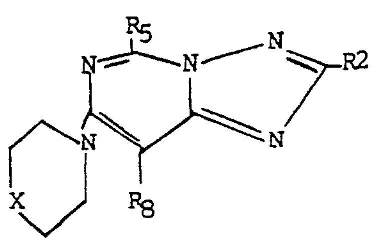

dans laquelle $R_2$ représente l'hydrogène ou un radical alkyle de 1 à 4 atomes de carbone, $R_5$ représente l'hydrogène, un radical alkyle de 1 à 4 atomes de carbone ou le radical phényle; $R_8$ représente l'hydrogène, un radical alkyle de 1 à 4 atomes de carbone ou le radical phényle; et X représente l'oxygène, le soufre, le

radical sulfinyle, le radical sulfonyle, le radical méthylène, un radical imino ou un radical N-alkylimino de 1 à 4 atomes de carbone, comprenant le chauffage d'une triazolo[4,3-c]pyrimidine de la formule:

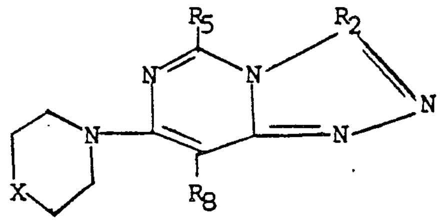

dans laquelle $R_2$, $R_5$, $R_8$ et X ont la définition donnée précédemment, en présence d'un acide aqueux pour former la triazolo[1,5-c]pyrimidine susdite.

2. Procédé suivant la revendication 1, caractérisé en ce que la triazolo[4,3-c]pyrimidine s'obtient par chauffage d'un composé de la formule:

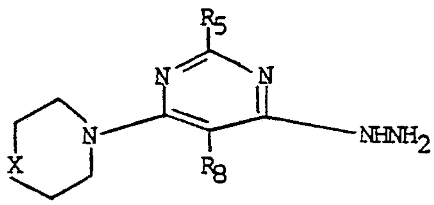

dans laquelle $R_5$, $R_8$ et X ont la définition donnée dans la revendication 1, en présence d'un orthoester de trialkyle de la formule:

$$R_2C(OAlk)_3$$

où $R_2$ et la définition donnée dans la revendication 1 et Alk désigne un radical alkyle de 1 à 4 atomes de carbone.

3. Procédé de préparation d'une triazolo[1,5-c]pyrimidine de la formule:

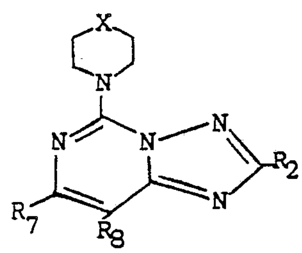

dans laquelle $R_2$ et $R_7$ représentent chacun de l'hydrogène ou un radical alkyle de 1 à 4 atomes de carbone, et $R_8$ et X ont la définition donnée dans la revendication 1, caractérisé en ce qu'il comnprend le chauffage d'une triazolo[4,3-c]pyrimidine de la formule:

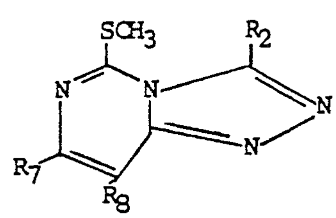

dans laquelle $R_2$, $R_7$ et $R_8$ ont la définition donnée précédemment, en présence d'un excès d'une amine hétérocyclique de la formule:

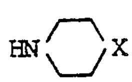

dans laquelle X a la définition donnée précédemment.

4. Procédé de fabrication d'un médicament, caractérisé en ce qu'il comprend la préparation d'une triazolo-pyrimidine par un procédé suivant l'une quelconque des revendications 1 à 3, et la mise en association de la triazolo-pyrimidine résultante avec un véhicule acceptable en pharmacie.

5. Utilisation d'un procédé suivant l'une quelconque des revendications 1 à 3 en vue de la fabrication d'un médicament destiné à provoquer une bronchodilatation chez les mammifères.